# EUROPEAN PATENT SPECIFICATION

(11) **EP 2 598 885 B1**
(45) Date of publication and mention of the grant of the patent: **20.07.2016**
(21) Application number: 11813229.9
(22) Date of filing: 29.07.2011
(51) Int. Cl.: G01N 33/573

(54) **BIOMARKER ASSAYS FOR DETECTING OR MEASURING INHIBITION OF TOR KINASE ACTIVITY**
BIOMARKER-ASSAYS ZUM NACHWEIS ODER ZUR MESSUNG EINER TOR-KINASE-AKTIVITÄTS-HEMMUNG
ANALYSES DE MARQUEURS BIOLOGIQUES POUR DÉTECTER OU MESURER L'INHIBITION DE L'ACTIVITÉ D'UNE KINASE TOR

(30) Priority: 30.07.2010 US 369455 P
(43) Date of publication of application: 05.06.2013
(73) Proprietor: Signal Pharmaceuticals, LLC, San Diego, CA 92121 (US)
(72) Inventor: WONG, Lilly, Solana Beach CA 92075 (US); XU, Shuichan, San Diego CA 92129 (US); DING, Jian-Hua, San Diego CA 92122 (US)
(74) Representative: Jones Day
(86) International application number: PCT/US2011/045842
(87) International publication number: WO 2012/016113

(56) References cited:
- US-A1- 2006 216 776
- US-A1- 2007 149 521
- US-A1- 2009 023 724
- US-A1- 2009 215 812
- US-A1- 2009 215 812

## Description

### 1. FIELD

Provided herein are methods for detecting and/or measuring the inhibition of TOR kinase activity in a subject and uses associated therewith.

### 2. BACKGROUND

The connection between abnormal protein phosphorylation and the cause or consequence of diseases has been known for over 20 years. Accordingly, protein kinases have become a very important group of drug targets. *See* Cohen, Nature Reviews Drug Discovery, 1:309-315 (2002). Various protein kinase inhibitors have been used clinically in the treatment of a wide variety of diseases, such as cancer and chronic inflammatory diseases, including diabetes and stroke. See Cohen, Eur. J. Biochem., 268:5001-5010 (2001), Protein Kinase Inhibitors for the Treatment of Disease: The Promise and the Problems, Handbook of Experimental Pharmacology, Springer Berlin Heidelberg, 167 (2005).

The protein kinases are a large and diverse family of enzymes that catalyze protein phosphorylation and play a critical role in cellular signaling. Protein kinases may exert positive or negative regulatory effects, depending upon their target protein. Protein kinases are involved in specific signaling pathways which regulate cell functions such as, but not limited to, metabolism, cell cycle progression, cell adhesion, vascular function, apoptosis, and angiogenesis. Malfunctions of cellular signaling have been associated with many diseases, the most characterized of which include cancer and diabetes. The regulation of signal transduction by cytokines and the association of signal molecules with protooncogenes and tumor suppressor genes have been well documented. Similarly, the connection between diabetes and related conditions, and deregulated levels of protein kinases, has been demonstrated. *See e.g.,* Sridhar et al. Pharmaceutical Research, 17(11):1345-1353 (2000). Viral infections and the conditions related thereto have also been associated with the regulation of protein kinases. Park et al. Cell 101 (7): 777-787 (2000).

Protein kinases can be divided into broad groups based upon the identity of the amino acid(s) that they target (serine/threonine, tyrosine, lysine, and histidine). For example, tyrosine kinases include receptor tyrosine kinases (RTKs), such as growth factors and non-receptor tyrosine kinases, such as the src kinase family. There are also dual-specific protein kinases that target both tyrosine and serine/threonine, such as cyclin *d*ependent kinases (CDKs) and mitogen-activated protein kinases (MAPKs).

Because protein kinases regulate nearly every cellular process, including metabolism, cell proliferation, cell differentiation, and cell survival, they are attractive targets for therapeutic intervention for various disease states. For example, cell-cycle control and angiogenesis, in which protein kinases play a pivotal role are cellular processes associated with numerous disease conditions such as but not limited to cancer, inflammatory diseases, abnormal angiogenesis and diseases related thereto, atherosclerosis, macular degeneration, diabetes, obesity, and pain.

Protein kinases have become attractive targets for the treatment of cancers. Fabbro et al., Pharmacology & Therapeutic 93:79-98 (2002). It has been proposed that the involvement of protein kinases in the development of human malignancies may occur by:
(1) genomic rearrangements (e.g., BCR-ABL in chronic myelogenous leukemia), (2) mutations leading to constitutively active kinase activity, such as acute myelogenous leukemia and gastrointestinal tumors, (3) deregulation of kinase activity by activation of oncogenes or loss of tumor suppressor functions, such as in cancers with oncogenic RAS, (4) deregulation of kinase activity by over-expression, as in the case of EGFR and (5) ectopic expression of growth factors that can contribute to the development and maintenance of the neoplastic phenotype. Fabbro et al., Pharmacology Therapeutic 93:79-98 (2002).

The elucidation of the intricacy of protein kinase pathways and the complexity of the relationship and interaction among and between the various protein kinases and kinase pathways highlights the importance of developing pharmaceutical agents capable of acting as protein kinase modulators, regulators or inhibitors that have beneficial activity on multiple kinases or multiple kinase pathways. Accordingly, there remains a need for new kinase modulators.

The protein named mTOR (mammalian target of rapamycin), which is also called FRAP, RAFTI or RAPT1), is a 2549-amino acid Ser/Thr protein kinase, that has been shown to be one of the most critical proteins in the mTOR/PI3K/Akt pathway that regulates cell growth and proliferation. Georgakis and Younes Expert Rev. Anticancer Ther. 6(1):131-140 (2006). mTOR exists within two complexes, mTORC1 and mTORC2. While mTORC1 is sensitive to rapamycin analogs (such as temsirolimus or everolimus), mTORC2 is largely rapamycin-insensitive. Notably, rapamycin is not a TOR kinase inhibitor. Several mTOR inhibitors have been or are being evaluated in clinical trials for the treatment of cancer. Temsirolimus was approved for use in renal cell carcinoma in 2007 and everolimus was approved in 2009 for renal cell carcinoma patients that have progressed on vascular endothelial growth factor receptor inhibitors. In addition, sirolimus was approved in 1999 for the prophylaxis of renal transplant rejection. The interesting but limited clinical success of these mTORC1 inhibitory compounds demonstrates the usefulness of mTOR inhibitors in the treatment of cancer and transplant rejection, and the increased potential for compounds with both mTORC1 and mTORC2 inhibitory activity.

Due to the potential pharmaceutical applications for inhibitors of TOR kinase activity, there is a need for methods for detecting and/or measuring the inhibition of TOR kinase activity *in vivo*.

Citation or identification of any reference in Section 2 of this application is not to be construed as an admission that the reference is prior art to the present application.

### 3. SUMMARY

Provided herein are in vitro methods for detecting or measuring the inhibition of TOR kinase activity in a subject, comprising the use of flow cytometry to measure the amount of phosphorylated 4EBP1 (also referred herein as "p4EBP1" in biological samples which have been obtained from said subject prior to and after the administration of a TOR kinase inhibitor, wherein the TOR kinase inhibitor is 7-(6-(2-hydroxypropan-2-yl)pyridin-3-yl)-1-(*trans-4-*methoxycyclohexyl)-3,4-dihydropyrazino[2,3-b]pyrazin-2(1H)-one or a pharmaceutically acceptable salt, clathrate, solvate, stereoisomer, tautomer, or prodrug thereof, wherein a decrease in the amount of the phosphorylated 4EBP1 confirms inhibition of TOR kinase activity in the subject.

Further provided herein are the above in vitro methods for determining a dose-response relationship for the administration of a TOR kinase inhibitor to a subject, wherein said subject is administered varying doses of said TOR kinase inhibitor and the amount of TOR kinase activity inhibition in said subject resulting from each dose of said TOR kinase inhibitor is determined using flow cytometry to measure the amount of phosphorylated 4EBP1 in biological samples which have been obtained from said subject prior to and after each administration of said TOR kinase inhibitor.

Further provided herein are the above in vitro methods for determining whether a subject is sensitive to a TOR kinase inhibitor, comprising administering said subject said TOR kinase inhibitor and determining whether or not said TOR kinase activity is inhibited in said subject using flow cytometry to measure the amount of phosphorylated 4EBP1 in biological samples which have been obtained from said subject prior to and after the administration of said TOR kinase inhibitor.

Further provided herein are the above in vitro methods for determining the effective amount of a TOR kinase inhibitor for the treatment or management of a disease in a subject, comprising administering said subject varying doses of said TOR kinase inhibitor and determining the amount of TOR kinase activity inhibition in said patient resulting from each dose of said TOR kinase inhibitor using flow cytometry to measure the amount of phosphorylated 4EBP1 in biological samples which have been obtained from said patient prior to and after each administration of said TOR kinase inhibitor.

Further described herein are methods for treating or managing a disease associated with TOR kinase in a patient having a disease associated with TOR kinase, comprising administering to said patient an effective amount of a TOR kinase inhibitor, wherein the effective amount of said TOR inhibitor is determined using a method provided herein.

In certain embodiments, the methods provided herein are carried out by way of contacting a biological sample from a patient with a TOR kinase inhibitor *ex vivo.*

Further provided herein are kits comprising one or more containers illled with an p4EBP1 antibody, wherein the kit is used for measuring the amount of 4EBP1 in biological samples using flow cytometry, wherein the TOR kinase inhibitor is 7-(6-(2-hydroxypropan-2-yl)pyridin-3-yl)-1*-(trans-*4*-*methoxycyclohexyl)-3,4-dihydropyrazino[2,3-b]pyrazin-2(1H)-one or a pharmaceutically acceptable salt, clathrate, solvate, stereoisomer, tautomer, or prodrug thereof.

The present embodiments can be understood more fully by reference to the detailed description and examples, which are intended to exemplify non-limiting embodiments.

### 4. BRIEF DESCRIPTION OF THE DRAWINGS

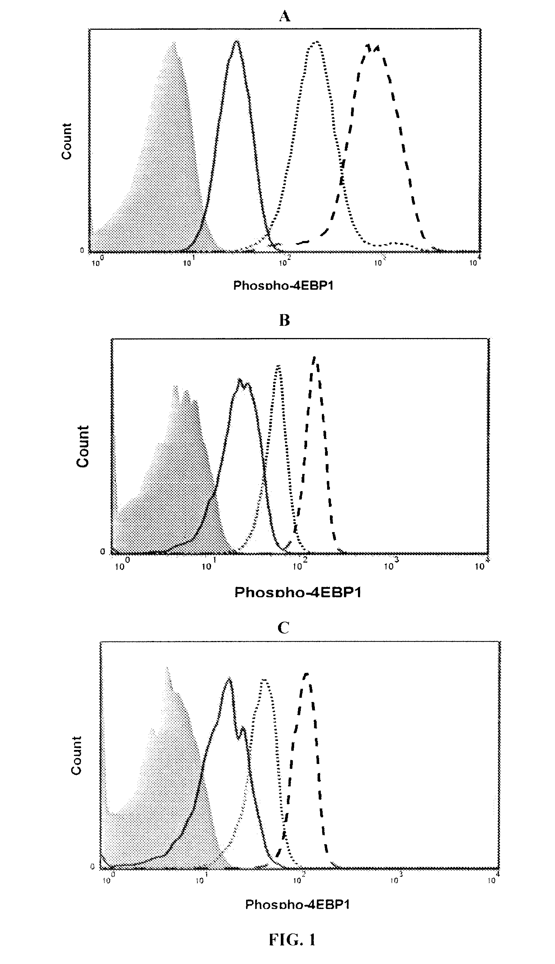

**FIG 1****.** Provides histograms representing cell populations of untreated cells (shaded), DMSO treated cells (dashed lined), specific blocking peptide treated cells (solid line), and Compound 1 treated cells (dotted line). (A) represents PC3 cells treated with 10 µM Compound 1. (B) Provides histograms representing CD91+ monocytes from whole blood of normal healthy volunteers treated *ex vivo* with 30 µM Compound 1. (C) Provides histograms representing CD91+ monocytes from whole blood of a lung cancer patient treated *ex vivo* with 30 µM Compound 1.

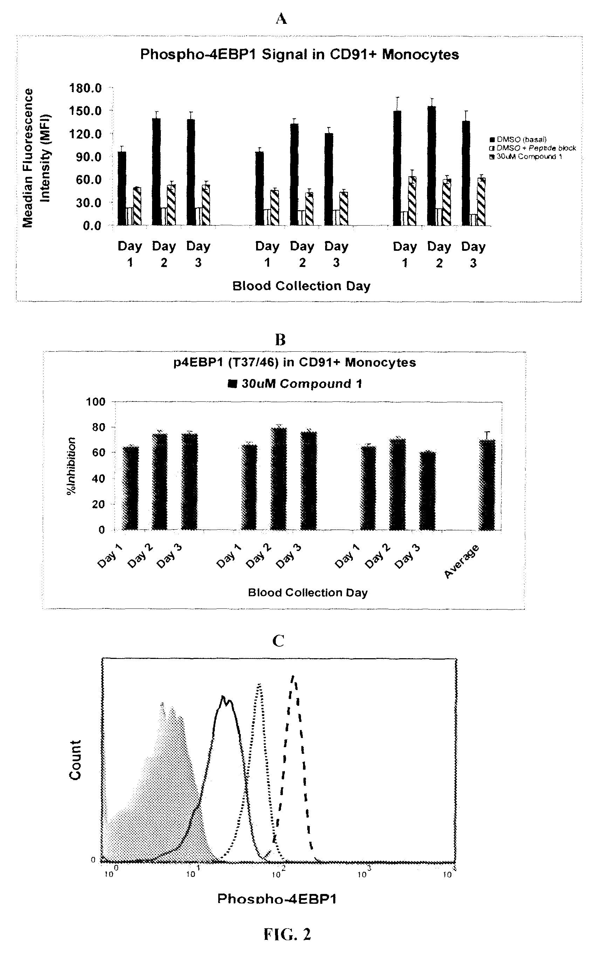

**FIG 2****.** Illustrates reproducibility of p4EBP1 assay with respect to day to day variation of p4EBP1 in monocytes of the same healthy donors (blood was drawn from 3 healthy donors on 3 consecutive days). (A) Illustrates mean fluorescence intensity for all 3 donors on different days. (B) Illustrates inhibition by Compound 1. (C) Provides an illustrative histogram representing cell populations of untreated cells (shaded), DMSO treated cells (dashed lined), specific blocking peptide treated cells (solid line), and Compound I treated cells (dotted line).

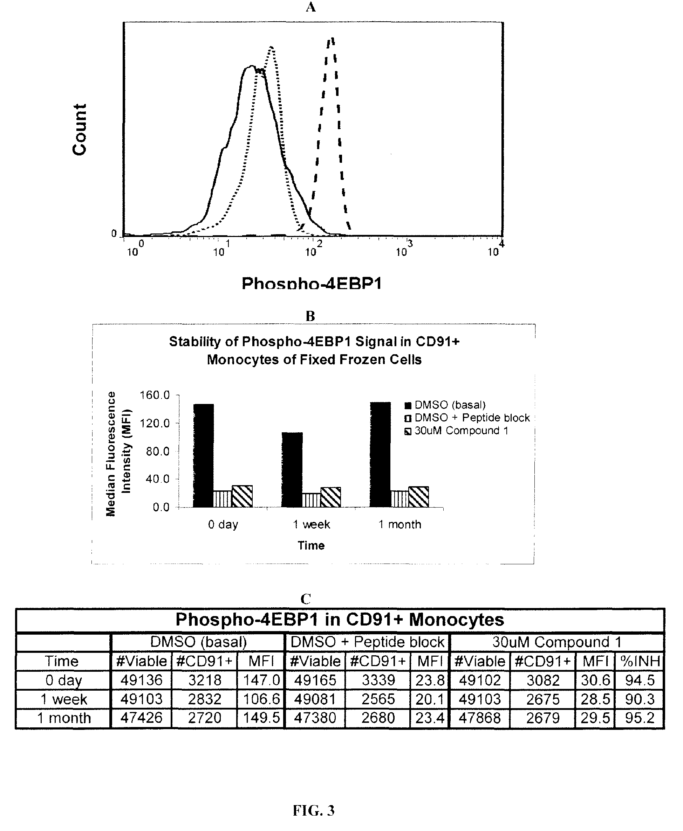

**FIG 3****.** Illustrates the stability of p4EBP1 signal in fixed frozen cells. All whole blood samples were treated with DMSO or Compound 1 at 37 °C for 2 hours and PBMCs were fixed and frozen without stabilization buffer. (A) Provides histograms of CD91+ monocytes from freshly prepared whole blood. (B) Provides a bar graph illustrating the stability of p4EBP1 signals in the CD91+ monoctye population of frozen cells over a period of 1 month. (C) Provides a table summarizing the viability and mean fluorescence intensity (MFI) of treated cells.

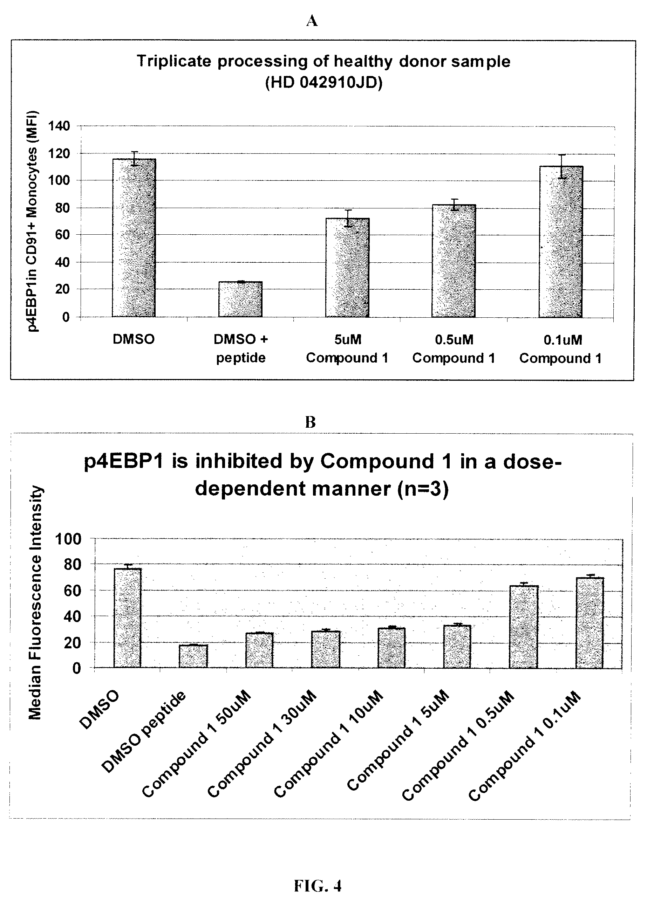

**FIG 4****.** (A) Illustrates *ex vivo* treatment of whole blood from healthy donor with Compound 1 for 2 hours at room temperature. Samples were processed in triplicate. Compound 1 treatment showed significant inhibition (p<0.005) at 5 µM and 0.5 µM. (B) Illustrates *ex vivo* treatment of whole blood from a healthy donor with Compound 1 at the indicated concentrations in triplicate for 2 hours at room temperature. Samples were processed in triplicate. Compound 1 treatment showed significant inhibition (p<0.005) at 5 µM and 0.5 µM.

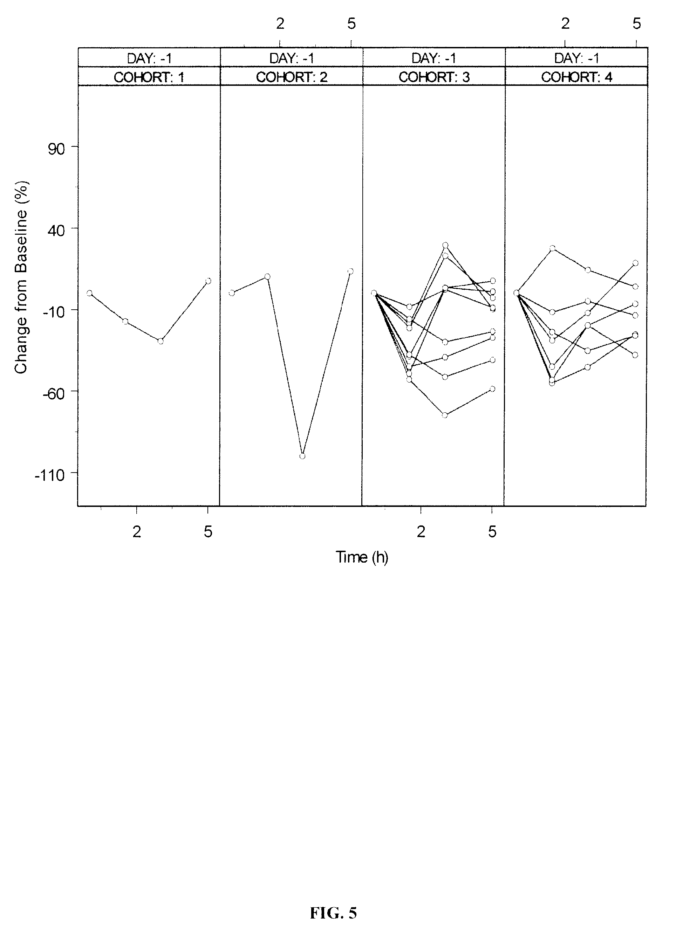

**FIG 5****.** Illustrates that p4EBP1 is inhibited in peripheral blood of patients. Each line represents a patient.

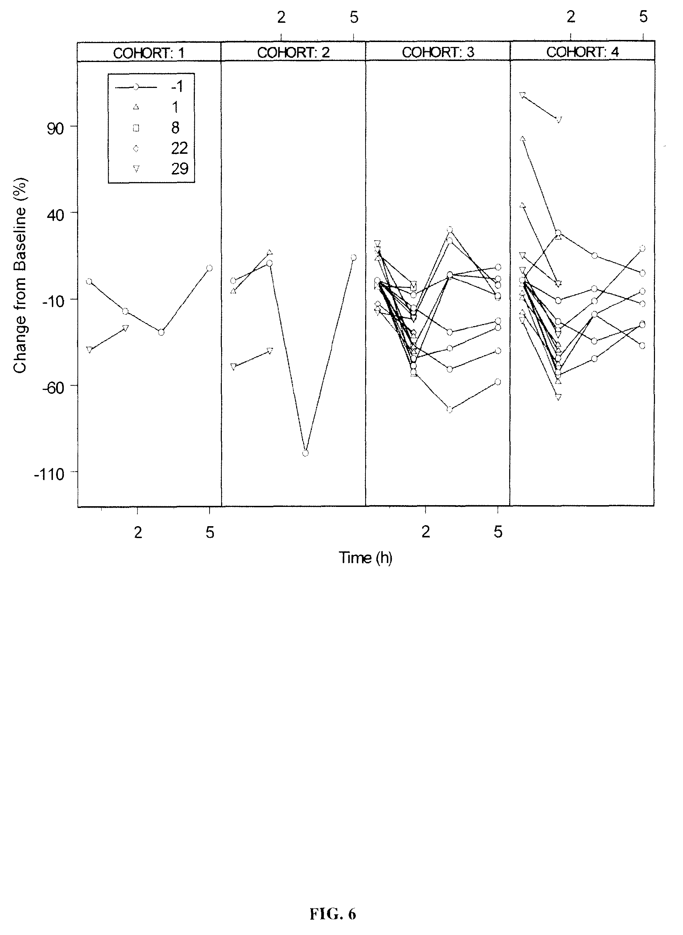

**FIG 6****.** Illustrates that p4EBP1 is inhibited in peripheral blood of patients. Each line represents a patient.

### 5. DETAILED DESCRIPTION

### 5.1 DEFINITIONS

The term "treating" as used herein means an alleviation, in whole or in part, of symptoms associated with a disorder or disease, or slowing, or halting of further progression or worsening of those symptoms.

The term "effective amount" in connection with an TOR kinase inhibitor means an amount capable of alleviating, in whole or in part, symptoms associated with a disease or disorder, or slowing or halting further progression or worsening of those symptoms. The effective amount of the TOR kinase inhibitor, for example in a pharmaceutical composition, may be at a level that will exercise the desired effect; for example, about 0.005 mg/kg of a subject's body weight to about 100 mg/kg of a patient's body weight in unit dosage for both oral and parenteral administration. As will be apparent to those skilled in the art, it is to be expected that the effective amount of a TOR kinase inhibitor disclosed herein may vary depending on the severity of the indication being treated.

The terms "patient" and "subject" as used herein include an animal, including, but not limited to, an animal such as a cow, monkey, horse, sheep, pig, chicken, turkey, quail, cat, dog, mouse, rat, rabbit or guinea pig, in one embodiment a mammal, in another embodiment a human. In one embodiment, a "patient" or "subject" is a human having a disease provided herein, such as a disease associated with a TOR kinase.

The term "biological sample" as used herein includes blood samples and tissue samples, such as tumor samples. In one embodiment, the biological sample is a peripheral blood sample. In another embodiment, the biological sample is plasma. In another embodiment, the biological sample is platelet rich plasma.

The term "TOR kinase inhibitor" as used herein means a compound (e.g. a small molecule) or a biologic (*e.g.*, a protein) capable of inhibition of TOR kinase activity [wherein the TOR kinase inhibitor is 7-(6-(2-hydroxypropan-2-yl)pyridin-3-yl)-1-(trans-4-methoxycyclohexyl)-3,4-dihydropyrazino[2,3-b]pyrazin-2(1H)-one] (*i.e., in vitro* or *in vivo*). As described herein, the TOR kinase inibitor is a compound disclosed in WO 2008/023161 (see, *e.g.,* page 5, line 5 to page 11, line 15), WO 2009/007751 (see, *e.g.,* page 9, line 8 to page 26, line 8), WO 2009/007749 (see, *e.g.,* page 9, line 21 to page 29, line 23), WO 2009/007750 (see, *e.g.,* page 9, line 21 to page 32, line 22), WO 2009/007748 (see, *e.g.,* page 9, line 6 to page 42, line 28), WO 2008/032028 (see, *e.g*., page 11, line 13 to page 21, line 13), WO 2008/032086 (see, *e.g.,* page 10 line 21 to page 15, line 22), WO 2008/032072 (see, *e.g.,* page 11, line 11 to page 16, line 13), WO 2008/032033 (see, *e.g.,* page 11, line 3 to page 16, line 5), WO 2008/032089 (see, *e.g*., page 11, line 11 to page 16, line 13), WO 2008/032060 (see, *e.g.,* page 11, line 3 to page 16, line 6), WO 2008/032091 (see, *e.g.,* page 11, line 11 to page 16, line 13), WO 2008/032036 (see, *e.g.,* page 11, line 13 to page 21, line 13), WO 2008/032077 (see, *e.g.,* page 10, line 21 to page 15, line 22), WO 2008/032064 (see, *e.g.*, page 11, line 3 to page 16, line 5). WO 2008/032027 (see, *e.g.,* page 10, line 21 to page 15, line 22), WO 2007/135398 (see, *e.g.,* page 11, line 28 to page 16, line 6), WO 2007/129052 (see, *e.g.,* page 10, line 8 to page 13, line 5), WO 2007/129044 (see, *e.g.,* page 10, line 22 to page 13, line 20), WO 2007/080382 (see. *e.g.,* page 9, line 20 to page 32, line 32), WO 2007/066102 (see, *e.g.,* page 9, line 22 to page 14, line 17), WO 2007/066099 (see, *e.g*., page 9, line 22 to page 14, line 14), WO 2007/066103 (see, *e.g.,* page 9, line 22 to page 14, line 16), WO 2007/060404 (see, *e.g.,* 5, line 4 to page 7, line 25), WO 2006/090169 (see, *e.g.,* page 4, lines 1-25), WO 2006/090167 (see, *e.g.,* page 3, line 33 to page 6, line 23), WO 2008/115974 (see, *e.g.,* page 4, paragraph [0012] to page 127, paragraph [0257]), WO 2009/052145 (see, *e.g.,* page 5, paragraph [0015] to page 81, paragraph [0082]), WO 2010/006072 (see, *e.g.,* page 28, line 1 to page 34, line 1), WO 2007/044698 (see, *e*.*g*., page 3, paragraph [0010] to the bottom of page 7), WO 2007/044813 (see, *e.g*., page 3, paragraph [0010] to the middle of page 7), WO 2007/044729 (see, *e*.*g*., page 3, paragraph [0010] to the bottom of page 10), WO 2007/129161 (see, *e.g.,* page 2, line 10 to page 9, line 19), WO 2006/046031 (see, *e.g.,* page 2, line 15 to page 4, line 12), WO 2003/072557 (see, *e.g.*, page 1, line 4 to page 2, line 27), WO 2004/048365 (see, *e.g.,* page 1, line 4 to page 4, line 17), WO 2004/078754 (see, *e*.*g*., page 1, line 4 to page 2, line 21), WO 2004/096797 (see, *e.g.,* page 1. line 4 to page 2, line 34), WO 2005/021519 (see, *e.g.,* page 1, line 4 to page 4, line 17) or US 2007/112005 (see, *e.g.,* page 2, paragraph [0012] to page 22, paragraph [0065]). TOR kinase inhibitors can be obtained via standard, well-known synthetic methodology, see *e.g.,* March, J. Advanced Organic Chemistry: Reactions Mechanisms, and Structure, 4th ed., 1992. Starting materials useful for preparing compounds of formula (III) and intermediates therefore, are commercially available or can be prepared from commercially available materials using known synthetic methods and reagents. Particular methods for preparing TOR kinase inhibitors are disclosed in U.S. Application No. 11/975,652, filed October 18, 2007, U.S. Application No. 11/975,657, tiled October 18, 2007 and U.S. Application No. 12/605,791, filed October 26, 2009. In a specific embodiment, the TOR kinase inhibitors do not include rapamycin or rapamycin analogs (rapalogs).

### 5.2 METHODS OF USE

Without being limited by theory, it is believed that because 4EBP1 (4E-binding protein 1, also referred to herein as "p4EBP1") is a direct substrates of TOR kinase (*i.e.,* TOR kinase catalyzes the phosphorylation of 4EBP1 to p4EBP1) the inhibition of TOR kinase activity *in vivo* can be measured by determining a subject's p4EBP1 levels (*e.g.,* levels in a biological sample from a subject) pre- and post-treatment with a TOR kinase inhibitor. In the following, the TOR kinase inhibitor is [7-(6-(2-hydroxypropan-2-yl)pyridin-3-yl)-1-(trans-4-methoxycyclohexyl)-3,4-dihydropyrazino[2,3-b]pyrazin-2(1H)-one].

Accordingly, provided herein are in vitro methods for detecting or measuring the inhibition of TOR kinase activity in a subject, comprising measuring the amount of p4EBP1 in a biological sample from said subject prior to and after the administration of the TOR kinase inhibitor. In one embodiment, the amount of p4EBP1 is measured using flow cytometry.

In one embodiment, the amount of p4EBP1 in whole blood from a subject is measured. In another embodiment, the amount of p4EBP1 in peripheral blood mononuclear cells (PBMCs) from a subject is measured. In another embodiment, the amount of p4EBP1 in a tissue sample from a subject is measured. In another embodiment, the amount of p4EBP1 in a tumor sample from a subject is measured.

Further provided herein are in vitro methods for determining a dose-response relationship for the administration of the TOR kinase inhibitor to a subject, wherein said subject is administered varying doses of said TOR kinase inhibitor and the amount of TOR kinase activity inhibition in said subject resulting from each dose of said TOR kinase inhibitor is determined using a method provided herein.

Further provided herein are in vitro methods for determining whether a subject is sensitive to a TOR kinase inhibitor, comprising administering said subject said TOR kinase inhibitor and determining whether or not TOR kinase activity is inhibited in said subject using a method provided herein.

Further provided herein are in vitro methods for determining the effective amount of the TOR kinase inhibitor for the treatment or management of a disease in a subject, comprising administering said subject varying doses of said TOR kinase inhibitor and determining the amount of TOR kinase activity inhibition in said patient resulting from each dose of said TOR kinase inhibitor using a method provided herein.

Further described herein are methods for treating or managing a disease associated with TOR kinase in a patient having a disease associated with TOR, comprising administering to said patient an effective amount of a TOR kinase inhibitor, wherein the effective amount of said TOR inhibitor is determined using a method provided herein.

In certain embodiments, the methods provided herein are carried out by way of contacting a biological sample from a patient with the TOR kinase inhibitor *ex vivo.* For example, instead of administration of the TOR kinase inhibitor to a subject, methods provided herein can comprise measuring the amount of p4EBP1 in a biological sample from a subject, contacting said biological sample with the TOR kinase inhibitor *ex vivo,* followed by measurement of the amount of p4EBP1 in said biological sample after said contacting. Accordingly, provided herein are methods for detecting or measuring the inhibition of TOR kinase activity in a biological sample from a subject, comprising measuring the amount of p4EBP1 in said biological sample prior to and after contacting said biological sample with the TOR kinase inhibitor *ex vivo.* The amount of p4EBP1 is measured using flow cytometry.

In certain embodiments, an IC₅₀ value of about 250 µM or less, about 100 µM or less, about 10 µM or less, about 1 µM or less or about 0.10 µM or less indicates that the TOR kinase inhibitor is effective for treating a disease associated with TOR kinase activity, such as a disease provided herein.

Further provided herein are kits comprising one or more containers filled with reagents for detecting p4EBP1 using flow cytometry and instructions for detecting p4EBP1 using flow cytometry. In one embodiment, the kits comprise one or more containers filled with an anti-p4EBP1 antibody. In certain embodiments, the anti-p4EBP1 antibody is Alexa Fluor 647 mouse anti-p4EBP1 or an anti-p4EBP1 antibody conjugated to Alexa Fluor 488. The kits provided herein further comprise the TOR kinase inhibitor provided herein.

In one embodiment, the patient has a solid tumor cancer. In another embodiment, the patient has a blood cancer. In a particular embodiment, the blood cancer is leukemia, such as chronic leukemia, acute leukemia, erythroleukemia, lymphocytic leukemia, myeloid leukemia or myelogenous leukemia. In another embodiment, the patient has a blood cancer other than lymphoblastic leukemia (e.g., T-cell acute lymphoblastic leukemia).

### 5.3 TOR KINASE INHIBITORS DESCRIBED HEREIN

TOR kinase inhibitors include, compounds having the following formula (I): 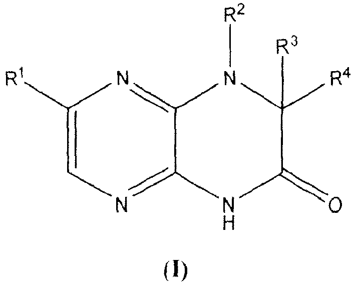 and pharmaceuticaly acceptable salts, clathrates, solvates, stereoisomers, tautomers, and prodrugs thereof, wherein:
R¹ is substituted or unsubstituted C₁₋₈ alkyl, substituted or unsubstituted aryl, substituted or unsubstituted cycloalkyl, substituted or unsubstituted heterocyclyl, or substituted or unsubstituted heterocyclylalkyl;
R² is II, substituted or unsubstituted C₁₋₈ alkyl, substituted or unsubstituted cycloalkyl, substituted or unsubstituted heterocyclyl, substituted or unsubstituted heterocyclylalkyl, substituted or unsubstituted aralkyl, or substituted or unsubstituted cycloalkylalkyl; and
R³ and R⁴ are each independently H, substituted or unsubstituted C₁₋₈ alkyl, substituted or unsubstituted aryl, substituted or unsubstituted cycloalkyl, substituted or unsubstituted heterocyclyl, substituted or unsubstituted heterocyclylalkyl, substituted or unsubstituted aralkyl, substituted or unsubstituted cycloalkylalkyl, or R³ and R⁴, together with the atoms to which they are attached, form a substituted or unsubstituted cycloalkyl or substituted or unsubstituted heterocyclyl;
or R² and one of R³ and R⁴, together with the atoms to which they are attached, form a substituted or unsubstituted heterocyclyl.

The TOR kinase inhibitor is not a compound depicted below, namely:

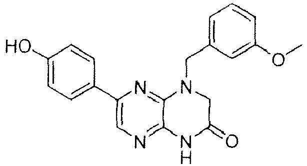

### 6-(4-hydroxyphenyl)-4-(3-methoxybenzyl)-3,4-dihydropyrazino[2,3-b]pyrazin-2(1H)-one;

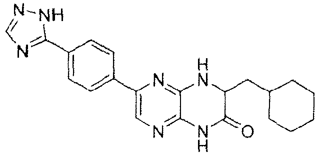

### 6-(4-(1H-1,2,4-triazol-5-yl)phenyl)-3-(cyclohexylmethyl)-3,4-dihydropyrazino[2,3-b]pyrazin-2(1H)-one; or,

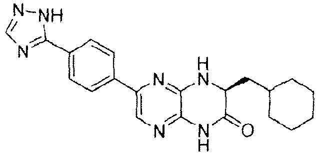

### (R)-6-(4-(1H-1,2,4-triazol-5-yl)phenyl)-3-(cyclohexylmethyl)-3,4-dihydropyrazino[2,3-b]pyrazin-2(H)-one.

The compounds of formula (I), R¹ is substituted or unsubstituted aryl or substituted or unsubstituted heteroaryl. As described herein, R¹ is phenyl, pyridyl, pyrimidyl, benzimidazolyl, indolyl, indazolyl, 1H-pyrrolo[2,3-b]pyridyl, 1H-imidazo[4,5-b]pyridyl, 1H-imidazo[4,5-b]pyridin-2(3H)-onyl, 3H-imidazo[4,5-b]pyridyl, or pyrazolyl, each optionally substituted. As described herein, R¹ is phenyl substituted with one or more substituents independently selected from the group consisting of substituted or unsubstituted C₁₋₈ alkyl (for example, methyl), substituted or unsubstituted heterocyclyl (for example, substituted or unsubstituted triazolyl or pyrazolyl), halogen (for example, fluorine), aminocarbonyl, cyano, hydroxyalkyl (for example, hydroxypropyl), and hydroxy. As described herein, R¹ is pyridyl substituted with one or more substituents independently selected from the group consisting of substituted or unsubstituted C₁₋₈ alkyl, substituted or unsubstituted heterocyclyl (for example, substituted or unsubstituted triazolyl), halogen, aminocarbonyl, cyano, hydroxyalkyl, -OR, and -NR₂, wherein each R is independently H, or a substituted or unsubstituted C₁₋₄ alkyl. As described herein, R¹ is 1H-pyrrolo[2,3-b]pyridyl or benzimidazolyl, each optionally substituted with one or more substituents independently selected from the group consisting of substituted or unsubstituted C₁₋₈ alkyl, and -NR₂, wherein each R is independently H, or a substituted or unsubstituted C₁₋₄ alkyl. As described herein,

As described herein, R¹ is 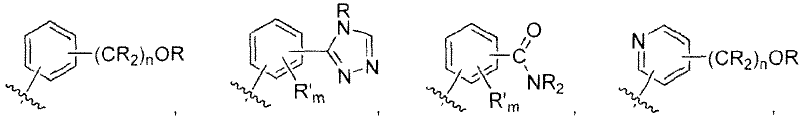 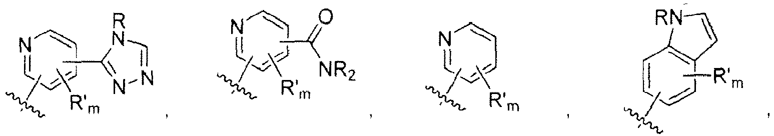 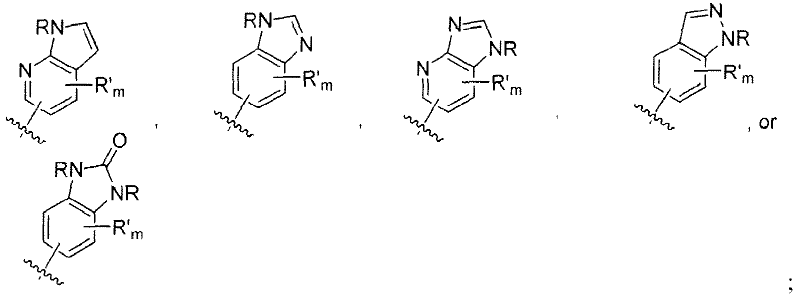 wherein R is at each occurrence independently H, or a substituted or unsubstituted alkyl (for example, methyl): R' is at each occurrence independently a substituted or unsubstituted C₁₋₄ alkyl, halogen (for example, fluorine), cyano, -OR, or -NR₂; m is 0-3; and n is 0-3. It will be understood by those skilled in the art that any of the subsitutuents R' may be attached to any suitable atom of any of the rings in the fused ring systems. It will also be understood by those skilled in the art that the connecting bond of R¹ (designated by the bisecting wavy line) may be attached to any of the atoms in any of the rings in the fused ring systems.

As described herein R¹ is 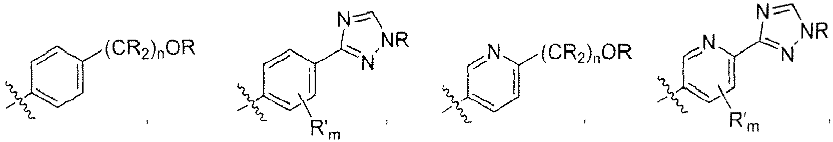 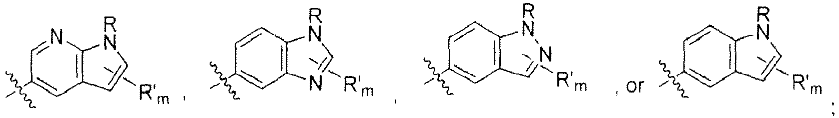 wherein R is at each occurrence independently H, or a substituted or unsubstituted C₁₋₄ alkyl; R' is at each occurrence independently a substituted or unsubstituted C₁₋₄ alkyl, halogen, cyano, -OR, or -NR₂; m is 0-3; and n is 0-3.

As described herein, R² is H, substituted or unsubstituted C₁₋₈ alkyl, substituted or unsubstituted cycloalkyl, substituted or unsubstituted heterocyclyl, substituted or unsubstituted C₁₋₄ alkyl-heterocyclyl, substituted or unsubstituted C₁₋₄ alkyl-aryl, or substituted or unsubstituted C₁₋₄ alkyl-cycloalkyl. For example, R² is H, methyl, ethyl, n-propyl, isopropyl, n-butyl, *sec*-butyl, isobutyl, *tert-*butyl*,* n-pentyl, isopentyl, cyclopentyl, cyclohexyl, tetrahydrofuranyl, tetrahydropyranyl, (C₁₋₄ alkyl)-phenyl, (C₁₋₄ alkyl)-cyclopropyl. (C₁₋₄ alkyl)-cyclobutyl, (C₁₋₄ alkyl)-cyclopentyl, (C₁₋₄ alkyl)-cyclohexyl, (C₁₋₄ alkyl)-pyrrolidyl, (C₁₋₄ alkyl)-piperidyl, (C₁₋₄ alkyl)-piperazinyl, (C₁₋₄ alkyl)-morpholinyl, (C₁₋₄ alkyl)-tetrahydrofuranyl, or (C₁₋₄ alkyl)-tetrahydropyranyl, each optionally substituted.

As described herein, R² is H, C₁₋₄ alkyl, (C₁₋₄alkyl)(OR), 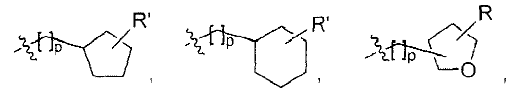 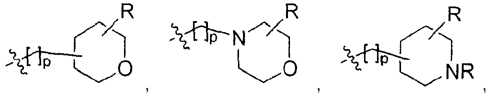 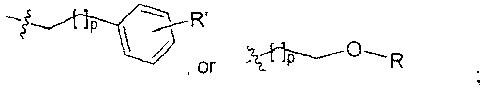 wherein R is at each occurrence independently H, or a substituted or unsubstituted C₁₋₄ alkyl (for example, methyl); R' is at each occurrence independently H, -OR, cyano, or a substituted or unsubstituted C₁₋₄ alkyl (for example, methyl); and p is 0-3.

As described herein, R² is H, C₁₋₄ alkyl, (C₁₋₄alkyl)(OR), 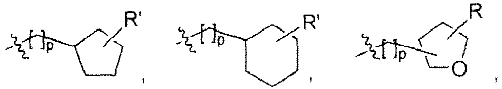 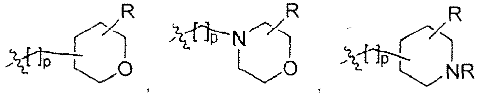 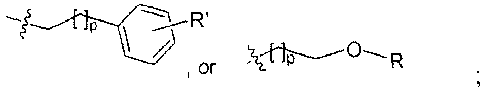 wherein R is at each occurrence independently H, or a substituted or unsubstituted C₁₋₂ alkyl; R' is at each occurrence independently H, -OR, cyano, or a substituted or unsubstituted C₁₋₂ alkyl; and p is 0-1.

As described herein, R² and one of R³ and R⁴ together with the atoms to which they are attached form a substituted or unsubstituted heterocyclyl. For example, the compound of formula (I) is 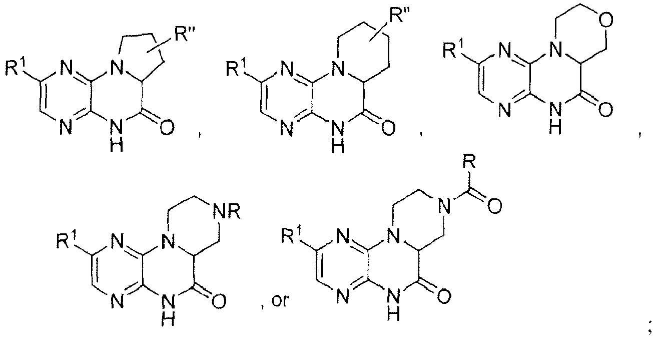 wherein R is at each occurrence independently H, or a substituted or unsubstituted C₁₋₄ alkyl; R" is H, OR. or a substituted or unsubstituted C₁₋₄ alkyl; and R¹ is as defined herein.

As defined herein, R³ and R⁴ are both H. In others, one of R³ and R⁴ is H and the other is other than H. Further, one of R³ and R⁴ is C₁₋₄ alkyl (for example, methyl) and the other is H. Further, both of R³ and R⁴ are C₁₋₄ alkyl (for example, methyl).

As described herein, R¹ is substituted or unsubstituted aryl, or substituted or unsubstituted heteroaryl. For example, R¹ is phenyl, pyridyl, pyrimidyl, benzimidazolyl, indolyl, indazolyl, 1 H-pyrrolo[2,3-b]pyridyl, 1 H-imidazo[4,5-b]pyridyl, 1H-imidazo[4,5-b]pyndin-2(3H)-onyl, 3H-imidazo[4,5-b]pyridyl, or pyrazolyl, each optionally substituted. As described herein, R¹ is phenyl substituted with one or more substituents independently selected from the group consisting of substituted or unsubstituted C₁₋₈ alkyl, substituted or unsubstituted heterocyclyl, halogen, aminocarbonyl, cyano, hydroxyalkyl and hydroxy. R¹ is pyridyl substituted with one or more substituents independently selected from the group consisting of cyano, substituted or unsubstituted C₁₋₈ alkyl, substituted or unsubstituted heterocyclyl, hydroxyalkyl, halogen, aminocarbonyl, -OR, and -NR₂, wherein each R is independently H, or a substituted or unsubstituted C₁₋₄ alkyl. Further, R¹ is 1H-pyrrolo[2,3-b]pyridyl or benzimidazolyl, optionally substituted with one or more substituents independently selected from the group consisting of substituted or unsubstituted C₁₋₈ alkyl, and -NR₂, wherein R is independently H, or a substituted or unsubstituted C₁₋₄alkyl

As described herein, the compounds of formula (I) have an R group set forth herein and an R² group set forth herein.

The compounds of formula (I) may be
6-(1H-pyrrolo[2,3-b]pyridin-3-yl)-4-(2-(tetrahydro-2H-pyran-4-yl)ethyl)-3,4-dihydropyrazino[2,3-b]pyrazin-2(1H)-one;
6-(4-methyl-6-(1H-1,2,4-triazol-3-yl)pyridin-3-yl)-4-((tetrahydro-2H-pyran-4-yl)methyl)-3,4-dihydropyrazmo[2,3-b]pyrazin-2(1H)-one;
6-(5-fluoro-2-methyl-4-(1H-1,2,4-triazol)-3-yl)phenyl)-4-((*trans*-4-methoxycyclohexyl)methyl)-3,4-dihydropyrazino[2,3-b]pyrazin-2(1H)-one;
6-(5-fluoro-2-methyl-4-(1H-1,2,4-triazol-3-yl)phenyl*)-*4-((*cis-*4-methoxycyclohexyl)methyl)-3,4-dihydropyrazino[2,3-b]pyrazin-2(1H)-one;
6-(6-(1H-1,2,4-triazol-3-yl)pyridin-3-yl)-4-((*trans*-4-methoxycyclohexyl)methyl)-3,4-dihydropyrazino[2,3-b]pyrazin-2(1H)-one;
6-(5-fluoro-2-methyl-4-(1H-1,2,4-trtiazol-3-yl)phenyl)-4-((*trans*-4-hydroxycyclohexyl)methyl)-3,4-dihydropyrazino[2,3-b]pyrazin-2(1H)-one;
6-(-6-(1H-1,2,4-triazol-3-yl)pyridin-3-yl)-4-((*cis*-4-methoxycyclohexyl)methyl)-3,4-dihydropyrazino[2,3-b]pyrazin-2(1H)-one;
6-(6-(1H-1,2,4-triazol-3-yl)pyridin-3-yl)-4-((*trans*-4-hydroxycyclohexyl)methyl)-3,4-dihydropyrazino[2,3-b]pyrazin-2(1H)-one;
6-(6-(1H-1,2,4-triazol-3-yl)pyridin-3-yl)-4-(*cis*-4-hydroxycyclohexyl)-3,4-dihydropyrazino[2,3-b]pyrazin-2(1H)-one;
6-(6-(1H-1,2,4-triazol-3-yl)pyridin-3-yl)-4-((*cis*-4-hydroxycyclohexyl)methyl)-3,4-dihydropyrazino[2,3-b]pyrazin-2(1H)-one;
6-(5-fluoro-2-methyl-4-(1H-1,2,4-triazol-3-yl)phenyl)-4-(*trans*-4-methoxycyclohexyl)-3,4-dihydropyrazino[2,3-b]pyrazin-2(1H)-one;
6-(6-(1H-1,2,4-triazol-3-yl)pyridin-3-yl)-4-(*trans*-4-methoxycyclohexyl)-3,4-dihydropyrazino[2,3-b]pyrazin-2(1H)-one;
6-(6-(1H-1,2,4-triazol-3-yl)pyridin-3-yl)-4-(*trans-*4-hydroxycyclohexyl)-3,4-dihydropyrazino[2,3-b]pyrazin-2(1H)-one;
6-(5-fluoro-2-methyl-4-(1H-1,2,4-triazol-3-yl)phenyl)-4-((*cis*-4-hydroxycyclohexyl)methyl)-3,4-dihydropyrazino[2,3-b]pyrazin-2(1H)-one;
6-(6-(1H-1,2,4-triazol-3-yl)pyridin-3-yl)-4-(*cis*-4-methoxycyclohexyl)-3,4-dihydropyrazino[2,3-b]pyrazin-2(1H)-one;
6-(6-(1H-1,2,4-triazol-3-yl)pyridin-3-yl)-4-(2-methoxyethyl)-3,4-dihydropyrazino[2,3-b]pyrazin-2(1H)-one;
6-(6-(1H-1,2,4-triazol-3-yl)pyridin-3-yl)-4-isopropyl-3,4-dihydropyrazino[2,3-b]pyrazin-2(1H)-one;
6-(5-fluoro-2-methyl-4-(1H-1,2,4-triazol-3-yl)phenyl)-4-(*cis*-4-hydroxycyclohexyl)-3,4 dihydropyrazino[2,3-b]pyrazin-2(1H)-one;
6-(5-fluoro-2-methyl-4-(1H-1,2,4-triazol-3-yl)phenyl)-4-(*cis*-4-methoxycyclohexyl)-3,4-dihydropyrazino[2,3-b]pyrazin-2(1H)-one;
6-(5-fluoro-2-methyl-4-(1H-1,2,4-triazol-3-yl)phenyl)-4-(2-methoxyethyl)-3,4-dihydropyrazino[2,3-b]pyrazin-2(1H)-one;
6-(6-(1H-1,2,4-triazol-3-yl)pyridin-3-yl)-4-(tetrahydro-2H-pyran-4-yl)-3,4-dihydropyrazino[2,3-b]pyrazin-2(1H)-one;
6-(6-(1H-1,2,4-triazol-3-yl)pyridin-3-yl)-4-ethyl-3,4-dihydropyrazino[2,3-b]pyrazin-2(1H)-one;
6-(5-fluoro-2-methyl-4-(1H-1,2,4-triazol-3-yl)phenyl)-4-(*trans*-4-hydroxycycloheyl)-3,4-dihydropyrazino[2,3-b]pyrazin-2(1H)-one;
6-(5-fluoro-2-methyl-4-(1H-1,2,4-triazol-3-yl)phenyl)-4-(tetrahydro-2H-pyran-4-yl)-3,4-dihydropyrazino[2,3-b]pyrazin-2(1H)-one;
6-(5-fluoro-2-methyl-4-(1H-1,2,4-triazol-3-yl)phenyl)-4-isopropyl-3,4-dihydropyrazino[2,3-b]pyrazin-2(1H)-one;
4-ethyl-6-(5-fluoro-2-methyl-4-(1H-1,2,4-triazol-3-yl)phenyl)-3,4-dihydropyrazino[2,3-b]pyrazin-2(1H)-one;
6-(3-fluoro-2-methyl-4-(1H-1,2,4-triazol-3-yl)phenyl)-4-(tetrahydro-2H-pyran-4-yl)-3,4-dihydropyrazino[2,3-b]pyrazin-2(1H)-one;
6-(3-fluoro-2-methyl-4-(1H-1,2,4-triazol-3-yl)phenyl)-4-(*cis*-4-methoxycyclohexyl)-3,4-dihydropyrazino[2,3-b]pyrazin-2(1H)-one;
6-(3-fluoro-2-methyl-4-(1H-1,2,4-triazol-3-yl)phenyl)-4-(*trans*-4-methoxycyclohexyl)-3,4-dihydropyrazino[2,3-b]pyrazin-2(1H)-one;
4-(2-methoxyethyl)-6-(4-methyl-6-(1H-1,2,4-triazol-3-yl)pyridin-3-yl)-3,4-dihydropyrazino[2,3-b]pyrazin-2(1H)-one;
6-(3-(1H-1,2,4-triazol-5-yl)phenyl)-4-(2-(tetrahydro-2H-pyran-4-yl)ethyl)-3,4-dihydropyrazino[2,3-b]pyrazin-2(1H)-one;
5-(8-(2-methoxyethyl)-6-oxo-5,6,7,8-tetrahydropyrazino[2,3-b]pyrazin-2-yl)-4-methylpicolinamide;
3-(6-oxo-8-(2-(tetrahydro-2H-pyran-4-yl)ethyl)-5,6,7,8-tetrahydropyrazino[2,3-b]pyrazin-2-yl)benzamide;
3-(6-oxo-8-(2-(tetrahydro-2H-pyran-4-yl)ethyl)-5,6,7,8-tetrahydropyrazino[2,3-b]pyrazin-2-yl)benzonitrile;
5-(8-(*trans*-4-methoxycyclohexyl)-6-oxo-5,6,7,8-tetrahydropyrazino[2,3-b]pyrazin-2-yl)-4-methylpicolinamide;
6-(1H-imidazo[4,5-b]pyridin-6-yl)-4-(2-(tetrahydro-2H-pyran-4-yl)ethyl)-3,4-dihydropyrazino[2,3-b]pyrazin-2(1H)-one;
6-(1H-indazol-6-yl)-4-(2-(tetrahydro-2H-pyran-4-yl)ethyl)-3,4-dihydropyrazino[2,3-b]pyrazin-2(1H)-one;
4-((1R,3S)-3-methoxycyclopentyl)-6-(2-methyl-6-(4H-1,2,4-triazol-3-yl)pyridin-3-yl)-3,4-dihydropyrazino[2,3-b]pyrazin-2(1H)-one;
4-((1S,3R)-3-methoxycyclopentyl)-6-(2-methyl-6-(4H-1,2,4-triazol-3-yl)pyridin-3-yl)-3,4-dihydropyrazino[2,3-b]pyrazin-2(1H)-one;
4-((1R,3R)-3-methoxycyclopentyl)-6-(2-methyl-6-(4H-1,2,4-triazol-3-yl)pyridin-3-yl)-3,4-dihydropyrazino[2,3-b]pyrazin-2(1H)-one;
4-((11S,3S)3-methoxycyclopentyl)-6-(2-methyl-6-(4H-1,2,4-triazol-3-yl)pyridin-3-yl)-3,4-dihydropyrazino[2,3-b]pyrazin-2(1H)-one;
4-ethyl-6-(2-methyl-6-(4H-1,2,4-triazol-3-yl)pyridin-3-yl)-3,4-dihydropyrazino[2,3-b]pyrazin-2(1H)-one;
6-(1H-pyrroto[2,3-b]pyridin-5-yl)-4-(2-(tetrahydro-2H-pyran-4-yl)-ethyl)-3,4-dihydropyrazino[2,3-b]pyrazin-2(1H)-one;
6-(1H-indol-6-yl)-4-(2-(tetrahydro-2H-pyran-4-yl)ethyl)-3,4-dihydropyrazino[2,3-b]pyrazin-2(1H)-one;
6-(1H-indol-5-yl)-4-(2-(tetrahydro-2H-pyran-4-yl)ethyl)-3,4-dihydropyrazino[2,3-b]pyrazin-2(1H)-one;
4-(((1R,3S)-3-methoxycyclopentyl)methyl)-6-(2-methyl-6-(4H-1,2,4-triazol-3-yl)pyridin-3-yl)-3,4-dihydropyrazino[2,3-b]pyrazin-2(1H)-one;
4-(((1S,3R)-3-methoxycyclopentyl)methyl)-6-(2-methyl-6-(4H-1,2,4-triazol-3-yl)pyridin-3-yl)-3,4-dihydropyrazino[2,3-b]pyrazin-2(1H)-one;
6-(3-fluoro-2-methyl-4-(4H-1,2,4-triazol-3-yl)phenyl)-4-(2-(tetrahydro-2H-pyran-4-yl)ethyl)-3,4-dihydropyrazino[2,3-b]pyrazin-2(1H)-one;
6-(3-fluoro-2-methyl-4-(4H-1,2,4-triazol-3-yl)phenyl)-4-(2-methoxyethyl)-3,4-dihydropyrazino[2,3-b]pyrazin-2(1H)-one:
3,3-dimethyl-6-(4-methyl-6-(4H-1,2,4-triazol-3-yl)pyridin-3-yl)-4-((tetrahydro-2H-pyran-4-yl)methyl)-3,4-dihydropyrazino[2,3-b]pyrazin-2(1H)-one;
6-(6-(2-hydroxypropan-2-yl)pyridin-3-yl)-4-((1R,3S)-3-methoxycyclopentyl)-3,4-dihydropyrazino[2,3-b]pyrazin-2(1H)-one;
6-(6-(2-hydroxypropan-2-yl)pyridin-3-yl)-4-((1S,3R)-3-methoxycyclopentyl)-3,4-dihydropyrazino[2,3-b]pyrazin-2(1H)-one;
6-(6-(2-hydroxypropan-2-yl)pyridin-3-yl)-4-(((1S,3S)-3-methoxycyclopentyl)methyl)-3,4-dihydropyrazino[2,3-b]pyrazin-2(1H)-one;
6-(6-(2-hydroxypropan-2-yl)pyridin-3-yl)-4-(((1R,3R)-3-methoxycyclopentyl)methyl)-3,4-dihydropyrazino[2,3-b]pyrazin-2(1H)-one;
6-(6-(2-hydroxypropan-2-yl)pyridin-3-yl)-4-((1S,3S)-3-methoxycyclopentyl)-3,4-dihydropyrazino[2,3-b]pyrazin-2(1H)-one;
6-(6-(2-hydroxypropan-2-yl)pyridin-3-yl)-4-((1R,3R)-3-methoxycyclopentyl)-3,4-dihydropyrazino[2,3-b]pyrazin-2(1H)-one;
6-(6-(2-hydroxypropan-2-yl)pyridin-3-yl)-4-(((1R,3S)-3-methoxycyclopentyl)methyl)-3,4-dihydropyrazino[2,3-b]pyrazin-2(1H)-one;
6-(6-(2-hydroxypropan-2-yl)pyridin-3-yl)-4-(((1S,3R)-3-methoxycyclopentyl)methyl)-3,4-dihydropyrazino[2,3-b]pyrazin-2(1H)-one;
6-(3-fluoro-4-(4H-1,2,4-triazol-3-yl)phenyl)-4-(2-methoxyethyl)-3,4-dihydropyrazino[2,3-b]pyrazin-2(1H)-one;
6-(3-fluoro-4-(4H-1,2,4-triazol-3-yl)phenyl)-4-(2-(tetrahydro-2H-pyran-4-yl)ethyl)-3,4-dihydropyrazino[2,3-b]pyrazin-2(1H)-one:
7'-(2-methyl(-4-(4H-1,2,4-triazol-3-yl)phenyl)-1'-((tetrahydro-2H-pyran-4-yl)methyl)-1'H-spiro[cyclopentane-1,2'-pyrazinol[2,3-b]pyrazin]-3'(4'H)-one;
7'-(2-methyl-4-(4H-1,2,4-triazol-3-yl)phenyl)-1'-((tetrahydro-2H-pyran-4-yl)methyl)-1'H-spiro[cyclobutane-1,2'-pyrazino[2,3-b]pyrazin]-3'(4'H)-one;
4-(cyclopropylmethyl)-6-(6-(2-hydroxypropan-2-yl)pyridin-3-yl)-3,4-dihydropyrazino[2,3-b]pyrazin-2(1H)-one;
7'-(2-methyl-4-(4H-1,2,4-triazol-3-yl)phenyl)-1'H-spiro[cyclopentane-1,2'-pyrazino[2,3-b]pyrazin]-3'(4'H)-one;
7'-(2-methyl-4-(4H-1,2,4-triazol-3-yl)phenyl)-1'H-spiro[cyctobutane-1,2'-pyrazino[2,3-b]pyrazin]-3'(4'H)-one;
7'-(2-methyl-4-(4H-1,2,4-triazol-3-yl)phenyl)-1'H-spirojcyclopropane-1,2'-pyrazino[2,3-b]pyrazin]-3'(4'H)-one;
(R)-6-(4-(4H-1,2,4-triazol-3-yl)phenyl)-4-((tetrahydrofuran-2-yl)methyl)-3,4-dihydropyrazino[2,3-b]pyrazin-2(1H)-one;
(S)-6-(4-(4H-1,2,4-triazol-3-yl)phenyl)-4-((tetrahydrofuran-2-yl)methyl)-3,4-dihydropyrazino[2,3-b]pyrazin-2(1H)-one;
6-(1H-indazol-5-yl)-4-(2-(tetrahydro-2H-pyran-4-yl)ethyl)-3,4-dihydropyrazino[2,3-b]pyrazin-2(1H)-one;
4-(6-oxo-8-(2-(tetrahydro-2H-pyran-4-yl)ethyl)-5,6,7,8-tetrahydropyrazino[2,3-b]pyrazin-2-yl)benzamide;
4-(2-methoxyethyl)-3,3-dimethyl-6-(2-methyl-4-(4H-1,2,4-triazol-3-yl)phenyl)-3,4-dihydropyrazino[2,3-b]pyrazin-2(H)-one;
4-ethyl-3,3-dimethyl-6-(2-methyl-4-(4H-1,2,4-triazol-3-yl)phenyl)-3,4-dihydropyrazino[2,3-b]pyrazin-2(1H)-one;
6-(2-methyl-4-(4H-1,2,4-triazol-3-yl)phenyl)-3,4-dihydropyrazino[2,3-b]pyrazin-2(1H)-one; 3,3-dimethyl-6-(2-methyl-6-(4H-1,2,4-triazol-3-yl)pyridin-3-yl)-4-((tetrahydro-2H-pyran-4-yl)methyl)-3,4-dihydropyrazino[2,3-b]pyrazin-2(1H)-one;
(R)-6-(6-(1-hydroxyethyl)pyridin-3-yl)-4-(2-(tetrahydro-2H-pyran-4-yl)ethyl)-3,4-dihydropyrazino[2,3-b]pyrazin-2(1H)-one;
3,3-dimethyl-6-(2-methyl-4-(4H-1,2,4-triazol-3-yl)phenyl)-4-((tetrahydro-2H-pyran-4-yl)methyl)-3,4-dihydropyrazino[2,3-b]pyrazin-2(1H)-one:
6-(6-(2-hydroxypropan-2-yl)-4-methylpyridin-3-yl)-4-(*trans-*4-methoxycyclohexyl)-3,4-dihydropyrazino[2,3-b]pyrazin-2(1H)-one;
6-(6-(2-hydroxypropan-2-yl)-4-methylpyridin-3-yl)-4-((tetrahydro-2H-pyran-4-yl)methyl)-3,4-dihydropyrazino[2,3-b]pyrazin-2(1H)-one;
3,3-dimethyl-6-(2-methyl-4-(4H-1,2,4-triazol-3-yl)phenyl)-3,4-dihydropyrazino[2,3-b]pyrazin-2(1H)-one;
3,3-dimethyl-6-(2-methyl-6-(4H-1,2,4-triazol-3-yl)pyridin-3-yl)-4-(2-(tetrahydro-2H-pyran-4-yl)ethyl)-3,4-dihydropyrazino[2,3-b]pyrazin-2(1H)-one;
6-(6-(2-hydroxypropan-2-yl)-2-methylpyridin-3-yl)-4-((tetrahydro-2H-pyran-4-yl)methyl)-3,4-dihydropyrazino[2,3-b]pyrazin-2(1H)-one;
6-(6-(2-hydroxypropan-2-yl)-2-methylpyridin-3-yl)-4-(*trans*-4-methoxycyclohexyl)-3,4-dihydropyrazino[2,3-b]pyrazin-2(1H)-one;
(S)-6-(6-(1-hydroxyethyl)pyridin-3-yl)-4-(2-(tetrahydro-2H-pyran-4-yl)ethyl)-3,4-dihydropyrazino[2,3-b]pyrazin-2(1H)-one;
3,3-dirnethyl-6-(2-methyl-4-(4H-1,2,4-triazol-3-yl)phenyl)-4-(2-(tetrahydro-2H-pyran-4-yl)ethyl)-3,4-dihydropyrazino[2,3-b]pyrazin-2(1H)-one;
6-(6-(2-hydroxypropan-2-yl)pyridin-3-yl)-3,3-dimethyl-4-(2-(tetrahydro-2H-pyran-4-yl)ethyl)-3,4-dihydropyrazino[2,3-b]pyrazin-2(1H)-one;
6-(4-(2-hydroxypropan-2-yl)phenyl)-4-(*trans*-4-methoxycyclohexyl)-3,4-dihydropyrazino[2,3-b]pyrazin-2(1H)-one;
6-(4-(2-hydroxypropan-2-yl)phenyl)-4-((*trans*-4-methoxycyclohexyl)methyl)-3,4-dihydropyrazino[2,3-b]pyrazin-2(1H)-one;
4-(*cis*-4-methoxycyclohexyl)-6-(2-methyl-6-(4H-1,2,4-triazol-3-yl)pyridin-3-yl)-3,4-dihydropyrazino[2,3-b]pyrazin-2(1H)-one;
4-(*trans*-4-methoxycyclohexyl)-6-(2-methyl-6-(4H-1,2,4-triazol-3-yl)pyridin-3-yl)-3,4-dihydropyrazino[2,3-b]pyrazin-2(1H)-one;
6-(4-(2-hydroxypropan-2-yl)phenyl)-4-((tetrahydro-2H-pyran-4-yl)methyl)-3,4-dihydropyrazino[2,3-b]pyrazin-2(1H)-one;
4-(2-methoxyethyl)-6-(2-methyl-6-(4H-1,2,4-triazol-3-yl)pyridin-3-yl)-3,4-dihydropyrazino[2,3-b]pyrazin-2(1H)-one;
9-(6-(4H-1,2,4-triazol-3-yl)-3-pyridyl)-6,11,4a-trihydromorpholino[4,3-e]pyrazino[2,3-b]pyrazin-5-one;
6-(2-methyl-6-(4H-1,2,4-triazol-3-yl)pyridin-3-yl)-4-((tetrahydro-2H-pyran-4-yl)methyl)-3,4-dihydropyrazino[2,3-b]pyrazin-2(1H)-one;
5-(8-(*cic*-4-methoxycyclohexyl)-6-oxo-5,6,7,8-tetrahydropyrazino[2,3-b]pyrazin-2-yl)-6-methylpicolinonitrile;
6-(6-(4H-1,2,4-triazol-3-yl)pyridin-3-yl)-4-(2-(tetrahydro-2H-pyran-4-yl)ethyl)-3,4-dihydropyrazino[2,3-b]pyrazin-2(1H)-one;
9-(4-(4H-1,2.4-triazol-3-yl)-2-methylphenyl)-3-(2-methoxyacetyl)-6,11,4a-trihydropiperazino[1,2-e]pyrazino[2,3-b]pyrazin-5-one;
9-(4-(4H-1,2,4-triazol-3-yl)-2-methylphenyl)-6,11,4a-trihydropiperazino[1,2-e]pyrazino[2,3-b]pyrazin-5-one;
9-(4-(4H-1,2,4-triazol-3-yl)-2-methylphenyl)-3-(2-methoxyethyl)-6,11,4a-trihydropiperazino[1,2-e]pyrazino[2,3-b]pyrazin-5-one;
4-(cyclopentylmethyl)-6-(2-methyl-6-(4H-1,2,4-triazol-3-yl)pyridin-3-yl)-3,4-dihydropyrazino[2,3-b]pyrazin-2(1H)-one;
9-(6-(4H-1,2,4-triazol-3-yl)-2-methyl-3-pyridyl)-6,11,4a-trihydromorpholino[4,3-e]pyrazino[2,3-b]pyrazin-5-one;
4-(*trans*-4-hydroxycyclohexyl)-6-(6-(2-hydroxypropan-2-yl)pyridin-3-yl)-3,4-dihydropyrazino[2,3-b]pyrazin-2(1H)-one;
4-(*cis*-4-hydroxycyclohexyl)-6-(6-(2-hydroxypropan-2-yl)pyridin-3-yl)-3,4-dihydropyrazino[2,3-b]pyrazin-2(1H)-one;
6-(6-(2-hydroxypropan-2-yl)pyridin-3-yl)-4-((tetrahydrofuran-3-yl)methyl)-3,4-dihydropyrazino[2,3-b]pyrazin-2(1H)-one;
4-(cyclopentylmethyl)-6-(6-(2-hydroxypropan-2-yl)pyridin-3-yl)-3,4-dihydropyrazino[2,3-b]pyrazin-2(1H)-one:
6-(6-(2-hydroxypropan-2-yl)pyridin-3-yl)-4-neopentyl-3,4-dihydropyrazino[2,3-b]pyrazin-2(1H)-one;
6-(6-(2-hydroxypropan-2-yl)pyridin-3-yl)-4-isobutyl-3,4-dihydropyrazino[2,3-b]pyrazin-2(1H)-one;
3-methyl-6-(2-methyl-4-(4H-1,2,4-triazol-3-yl)phenyl)-4-(2-(tetrahydro-2H-pyran-4-yl)ethyl)-3,4-dihydropyrazino[2,3-b]pyrazin-2(1H)-one;
6-(6-(2-hydroxypropan-2-yl)pyridin-3-yl)-4-(piperidin-4-yl)-3,4-dihydropyrazino[2,3-b]pyrazin-2(1H)-one:
6-(6-(2-hydroxypropan-2-yl)pyridin-3-yl)-4-(2-(tetrahydro-2H-pyran-3-yl)ethyl)-3,4-dihydropyrazino[2,3-b]pyrazin-2(1H)-one;
8-(4-(4H-1,2,4-triazol-3-yl)-2-methylphenyl)(3aS,2R)-2-methoxy-5,10,3a-trinydropyrazino[2,3-b]pyrrolidino[1,2-e]pyrazin-4-one;
8-(4-(4H-1,2,4-triazol-3-yl)-2-methylphenyl)(2R,3aR)-2-methoxy-5,10,3a-trihydropyrazino[2,3-b]pyrrolidino[1,2-e]pyrazin-4-one;
8-(4-(4H-1,2,4-triazol-3-yl)-2-methylphenyl)(2S,3aR)-2-methoxy-5,10,3a-trihydropyrazino[2,3-b]pyrrolidino[1,2-e]pyrazin-4-one:
8-(4-(4H-1,2,4-triazol-3-yl)-2-methylphenyl)(2S,3aS)-2-methoxy-5,10,3a-trihydropyrazino[2,3-b]pyrrolidino[1,2-e]pyrazin-4-one;
6-(6-(2-hydroxypropan-2-yl)pyridin-3-yl)-4-(3-methoxypropyl)-3,4-dihydropyrazino[2,3-b]pyrazin-2(1H)-one;
(S)-6-(6-(2-hydroxypropan-2-yl)pyridin-3-yl)-4-((tetrahydrofuran-2-yl)methyl)-3,4-dihydropyrazino[2,3-b]pyrazin-2(1H)-one;
(R)-6-(6-(2-hydroxypropan-2-yl)pyridin-3-yl)-4-((tetrahydrofuran-2-yl)methyl)-3,4-dihydropyrazino[2,3-b]pyrazin-2(1H)-one;
6-(2-methyl-6-(4H-1,2,4-triazol-3-yl)pyridin-3-yl)-4-(2-(tetrahydro-2H-pyran-4-yl)ethyl)-3,4-dihydropyrazino[2,3-b]pyrazin-2(1H)-one;
9-(4-(4H-1,2,4-triazol-3-yl)-2-methylphenyl)-3-methyl-6,11,4a-trihydropiperazino[1,2-e]pyrazino[2,3-b]pyrazin-5-one;
9-(4-(4H-1,2,4-triazol-3-yl)phenyl)-6,11,4a-trihydromorpholino[4,3-e]pyrazino[2,3-b]pyrazin-5-one;
9-(4-(4H-1,2,4-triazol-3-yl)-2-methylphenyl)-6,11,4a-trihydropiperidino[1,2-e]pyrazino[2,3-b]pyrazin-5-one;
6-(6-(2-hydroxypropan-2-yl)pyridin-3-yl)-4-(*trans*-4-methoxycyclohexyl)-3,4-dihydropyrazino[2,3-b]pyrazin-2(1H)-one;
6-(6-(2-hydroxypropan-2-yl)pyridin-3-yl)-4-(*cis*-4-methoxycyclohexyl)-3,4-dihydropyrazino[2,3-b]pyrazin-2(1H)-one:
6-(6-(2-hydroxypropan-2-yl)pyridin-3-yl)-4-(2-morpholinoethyl)-3,4-dihydropyrazino[2,3-b]pyrazin-2(1H)-one;
6-(6-(2-hydroxypropan-2-yl)pyridin-3-yl)-4-phenethyl-3,4-dihydropyrazino[2,3-b]pyrazin-2(1H)-one;
6-(6-(2-hydroxypropan-2-yl)pyridin-3-yl)-4-(tetrahydro-2H-pyran-4-yl)-3,4-dihydropyrazino[2,3-b]pyrazin-2(1H)-one;
4-(cyclohexylmethyl)-6-(6-(2-hydroxypropan-2-yl)pyridin-3-yl)-3,4-dihydropyrazino[2,3-b]pyrazin-2(1H)-one;
6-(6-(2-hydroxypropan-2-yl)pyridin-3-yl)-4-((*trans*-4-methoxycyctohexyl)methyl)-3,4-dihydropyrazino[2,3-b]pyrazin-2(1H)-one;
6-(6-(2-hydroxypropan-2-yl)pyridin-3-yl)-4-((*cis*-4-methoxycyclohexyl)methyl)-3,4-dihydropyrazino[2,3-b]pyrazin-2(1H)-one;
(R)-6-(6-(2-hydroxypropan-2-yl)pyridin-3-yl)-4-(tetrahydrofuran-3-yl)-3,4-dihydropyrazino[2,3-b]pyrazin-2(1H)-one;
(S)-6-(6-(2-hydroxypropan-2-yl)pyridin-3-yl)-4-(tetrahydrofuran-3-yl)-3,4-dihydropyrazino[2,3-b]pyrazin-2(1H)-one;
6-(6-(2-hydroxypropan-2-yl)pyridin-3-yl)-4-phenyl-3,4-dihydropyrazino[2,3-b]pyrazin-2(1H)-one;
(S)-6-(6-(2-hydroxypropan-2-yl)pyridin-3-yl)-3-methyl-4-(2-(tetrahydro-2H-pyran-4-yl)ethyl)-3,4-dihydropyrazino[2,3-b]pyrazin-2(1H)-one;
9-[6-(1-hydroxy-isopropyl)-3-pyridyl]-6,11,4a-trihydromorpholino[4,3-e]pyrazino[2,3-b]pyrazin-5-one;
6-(6-(2-hydroxypropan-2-yl)pyridin-3-yl)-4-((tetrahydro-2H-pyran-4-yl)methyl)-3,4-dihydropyrazino[2,3-b]pyrazin-2(1H)-one;
6-(6-(2-hydroxypropan-2-yl)pyridin-3-yl)-4-(2-methoxyethyl)-3,4-dihydropyrazino[2,3-b]pyrazin-2(1 H)-one;
6-(2-amino-7-methyl-1H-benzo[d]imidazol-5-yl)-4-(3-(trifluoromethyl)benzyl)-3,4-dihydropyrazino[2,3-b]pyrazin-2(1H)-one;
6-(6-(2-hydroxypropan-2-yl)pyridin-3-yl)-4-(3-(trifluoromethyl)benzyl)-3,4-dihydropyrazino[2,3-b]pyrazin-2(1H)-one;
9-(4-(4H-1,2,4-triazol-3-yl)-2-methylphenyl)-6,11,4a-trihydromorpholino[4,3-e]pyrazino[2,3-b]pyrazin-5-one;
6-(4-methyl-2-(methylamino)-1H-benzo[d]imidazol-6-yl)-4-(2-(tetrahydro-2H-pyran-4-yl)ethyl)-3,4-dihydropyrazino[2,3-b]pyrazin-2(1H)-one;
8-(4-(4H-1,2,4-triazol-3-yl)-2-methylphenyl)-5,10,3a-trihydropyrazino[2,3-b]pyrrolidino[1,2-e]pyrazin-4-one,
6-(4-(4H-1,2,4-triazol-3-yl)phenyl)-4-ethyl-3,4-dihydropyrazino[2,3-b]pyrazin-2(1H)-one; 6-(4-(4H-1,2,4-triazol-3-yl)phenyl)-4-((tetrahydro-2H-pyran-4-yl)methyl)-3,4-dihydropyrazino[2,3-b]pyrazin-2(1H)-one;
6-(6-(2-hydroxypropan-2-yl)pyridin-3-yl)-4-(2-(tetrahydro-2H-pyran-4-yl)ethyl)-3,4-dihydropyrazino[2,3-b]pyrazin-2(1H)-one;
6-(4-(4H-1,2,4-triazol-3-yl)phenyl)-4-(2-methoxyethyl)-3,4-dihydropyrazino[2,3-b]pyrazin-2(1H)-one;
6-(4-(4H-1,2,4-triazol-3-yl)phenyl)-4-(3-(trifluoromethyl)benzyl)-3,4-dihydropyrazino[2,3-b]pyrazin-2(1H)-one;
6-(2-methyl-4-(4H-1,2,4-triazol-3-yl)phenyl)-4-(2-(tetrahydro-2H-pyran-4-yl)ethyl)-3,4-dihydropyrazino[2,3-b]pyrazin-2(1H)-one;
6-(4-methyl-1H-benzo[d]imidazol-6-yl)-4-(2-(tetrahydro-2H-pyran-4-yl)ethyl)-3,4-dihydropyrazino[2,3-b]pyrazin-2(1H)-one;
6-(4-(2-hydroxypropan-2-yl)phenyl)-4-(2-(tetrahydro-2H-pyran-4-yl)ethyl)-3,4-dihydropyrazino[2,3-b]pyrazin-2(1H)-one;
6-(4-(1H-1,2,4-triazol-5-yl)phenyl)-4-(2-(tetrahydro-2H-pyran-4-yl)ethyl)-3,4-dihydropyrazino[2,3-b]pyrazin-2(1H)-one; or a pharmaceutically acceptable salt thereof.

Further TOR kinase described herein are compounds having the following formula (II): 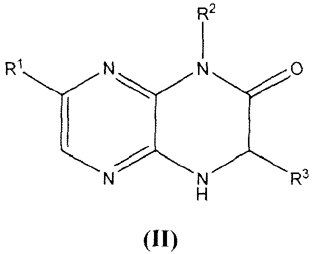 and pharmaceutically acceptable salts, clathrates, solvates, stereoisomers, tautomers. and prodrugs thereof, wherein:
R¹ is substituted or unsubstituted C₁₋₈ alkyl, substituted or unsubstituted aryl, substituted or unsubstituted cycloalkyl, substituted or unsubstituted heterocyclyl, or substituted or unsubstituted heterocyclylalkyl;
R² is H, substituted or unsubstituted C₁₋₈ alkyl, substituted or unsubstituted cycloalkyl, substituted or unsubstituted heterocyclyl, substituted or unsubstituted heterocyclylalkyl, substituted or unsubstituted aralkyl, or substituted or unsubstituted cycloalkylalkyl; and
R³ is H, or a substituted or unsubstituted C₁₋₈ alkyl.

As described herein, the TOR kinase inhibitor is not 7-(4-hydroxyphenyl)-1-(3-methoxybenzyl)-3,4-dihydropyrazino[2,3-b]pyrazin-2(1H)-one, depicted below:

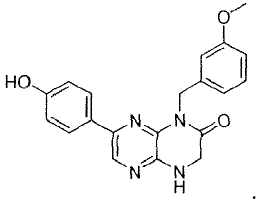

As described herein for compounds of formula (II), R is substituted or unsubstituted aryl or substituted or unsubstituted heteroaryl. For example, R¹ is phenyl, pyridyl, pyrimidyl, benzimidazolyl, 1H-pyrrolo[2,3-b]pyridyl, indazolyl, indolyl, 1H-imidazo[4,5-b]pyridyl, 1H-imidazo[4,5-b]pyridin-2(3H)-onyl, 3H-imidazo[4,5-b]pyridyl, or pyrazolyl, each optionally substituted. As described herein, R¹ is phenyl substituted with one or more substituents independently selected from the group consisting of substituted or unsubstituted C₁₋₈ alkyl (for example, methyl), substituted or unsubstituted heterocyclyl (for example, a substituted or unsubstituted triazolyl or pyrazolyl), aminocarbonyl, halogen (for example, fluorine), cyano, hydroxyalkyl and hydroxy. As described herein, R¹ is pyridyl substituted with one or more substituents independently selected from the group consisting of substituted or unsubstituted C₁₋₈ alkyl (for example, methyl), substituted or unsubstituted heterocyclyl (for example, a substituted or unsubstituted triazolyl), halogen, aminocarbonyl , cyano, hydroxyalkyl (for example, hydroxypropyl), -OR, and -NR₂, wherein each R is independently H, or a substituted or unsubstituted C₁₋₄ alkyl. As described herein, R¹ is 1H-pyrrolo[2,3-b]pyridyl or benzimidazolyl, optionally substituted with one or more substituents independently selected from the group consisting of substituted or unsubstituted C₁₋₈ alkyl, and -NR₂, wherein R is independently H, or a substituted or unsubstituted C₁₋₄ alkyl.

As described herein, R¹ is 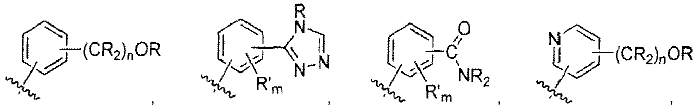 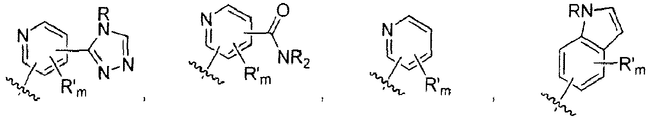 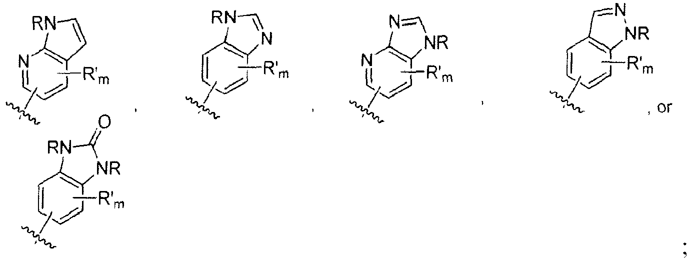 wherein R is at each occurrence independently H, or a substituted or unsubstituted C₁₋₄ alkyl (for example, methyl); R' is at each occurrence independently a substituted or unsubstituted C₁₋₄ alkyl (for example, methyl), halogen (for example, fluoro), cyano, -OR, or -NR₂; m is 0-3; and n is 0-3. It will be understood by those skilled in the art that any of the subsitutuents R' may be attached to any suitable atom of any of the rings in the fused ring systems.

As described herein for compounds of formula (II), R¹ is 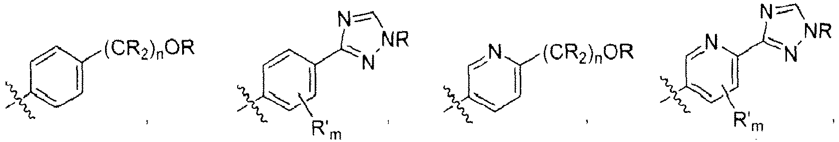 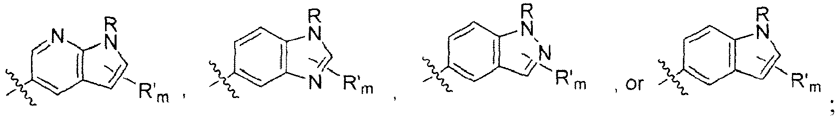 wherein R is at each occurrence independently H, or a substituted or unsubstituted C₁₋₄ alkyl; R' is at each occurrence independently a substituted or unsubstituted C₁₋₄ alkyl, halogen, cyano, -OR or -NR₂; m is 0-3; and n is 0-3.

As described herein of compounds of formula (II), R² is H, substituted or unsubstituted C₁₋₈ alkyl, substituted or unsubstituted cycloalkyl, substituted or unsubstituted heterocyclyl, substituted or unsubstituted C₁₋₄ alkyl-heterocyclyl, substituted or unsubstituted C₁₋₄ alkyl-aryl, or substituted or unsubstituted C₁₋₄ alkyl-cycloalkyl. For example, R² is H, methyl, ethyl, n-propyl, isopropyl, n-butyl, *sec*-butyl, isobutyl, *tert*-butyl, n-pentyl, isopentyl, cyclopentyl, cyclohexyl, tetrahydrofuranyl, tetrahydropyranyl, (C₁₋₄ alkyl)-phenyl, (C₁₋₄ alkyl)-cyclopropyl, (C₁₋₄ alkyl)-cyclobutyl, (C₁₋₄ alkyl)-cyctopentyl, (C₁₋₄ alkyl)-cyclohexyl, (C₁₋₄ alkyl)-pyrrolidyl, (C₁₋₄ alkyl)-piperidyl, (C₁₋₄ alkyl)-piperazinyl, (C₁₋₄ alkyl)-morpholinyl, (C₁₋₄ alkyl)-tetrahydrofuranyl, or (C₁₋₄ alkyl)-tetrahydropyranyl, each optionally substituted.

As described herein, R² is H, C₁₋₄ alkyl, (C₁₋₄alkyl)(OR), 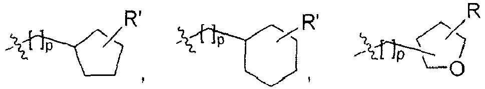 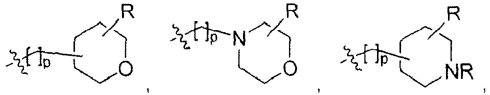 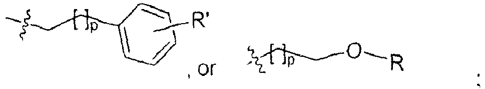 wherein R is at each occurrence independently H, or a substituted or unsubstituted C₁₋₄ alkyl (for example, methyl); R' is at each occurrence independently H, -OR, cyano,or a substituted or unsubstituted C₁₋₄ alkyl (for example, methyl); and p is 0-3.

As described herein for compounds of formula (II), R² is H, C₁₋₄ alkyl, (C₁₋₄alkyl)(OR), 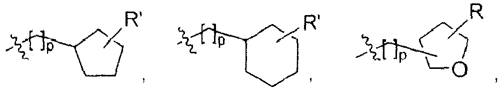 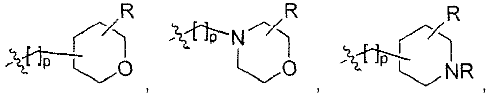 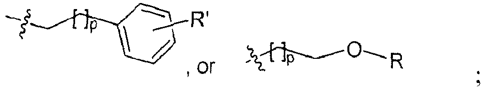 wherein R is at each occurrence independently H, or a substituted or unsubstituted C₁₋₂ alkyl; R' is at each occurrence independently H, -OR, cyano, or a substituted or unsubstituted C₁₋₂ alkyl; and p is 0-1.

As described herein for compounds of formula (II), R³ is H.

As described herein, R¹ is substituted or unsubstituted aryl, or substituted or unsubstituted heteroaryl. For example, R¹ is phenyl, pyridyl, pyrimidyl, benzimidazolyl, 1H-pyrrolo[2,3-b]pyridyl, indazolyl, indolyl, 1H-imidazo[4,5-b]pyridine, pyridyl, 1H-imidazo[4,5-b]pyridin-2(3H)-onyl, 3H-imidazo[4,5-b]pyridyl, or pyrazolyl, each optionally substituted. As described herein, R¹ is phenyl substituted with one or more substituents independently selected from the group consisting of substituted or unsubstituted C₁₋₈ alkyl, substituted or unsubstituted heterocyclyl, aminocarbonyl, halogen, cyano, hydroxyalkyl and hydroxy. Also, R¹ is pyridyl substituted with one or more substituents independently selected from the group consisting of C₁₋₈ alkyl, substituted or unsubstituted heterocyclyl, halogen, aminocarbonyl, cyano, hydroxyalkyl, -OR, and -NR₂, wherein each R is independently H, or a substituted or unsubstituted C₁₋₄ alkyl. Also, R¹ is 1H-pyrrolo[2,3-b]pyridyl or benzimidazolyl, optionally substituted with one or more substituents independently selected from the group consisting of substituted or unsubstituted C₁₋₈ alkyl, and -NR₂, wherein R is independently H, or a substituted or unsubstituted C₁₋₄ alkyl.

As described herein, the compounds of formula (II) have an R¹ group set forth herein and an R² group set forth herein.

As described herein for compounds of formula (II), the compound is
7-(5-fluoro-2-methyl-4-(1H-1,2,4-triazol-3-yl)phenyl)-1-((*trans*-4-methoxycyclohexyl)methyl)-3,4-dihydropyrazino[2,3-b]pyrazin-2(1H)-one;
7-(6-(1H-1,2,4-triazol-3-yl)pyridin-3-yl)-1-(*cis*-4-methoxycyclohexyl)-3,4-dihydropyrazino[2,3-b]pyrazin-2(1H)-one;
7-(1H-pyrrolo[2,3-b]pyridin-3-yl)-1-(2-(tetrahydro-2H-pyran-4-yl)ethyl)-3,4-dihydropyrazino[2,3-b]pyrazin-2(1H)-one;
7-(5-fluoro-2-methyl-4-(1H-1,2,4-triazol-3-yl)phenyl)-1-((*cis*-4-methoxycyclohexyl)methyl)-3,4-dihydropyrazino[2,3-b]pyrazin-2(1H)-one;
1-ethyl-7-(1H-pyrrolo[3,2-b]pyridin-5-yl)-3,4-dihydropyrazino[2,3-b]pyrazin-2(1H)-one; 7-(6-(1H-1,2,4-triazol-3-yl)pyridin-3-yl)-1-((*cis*-4-methoxycyclohexyl)methyl)-3,4-dihydropyrazino(2,3-b]pyrazin-2(1H)-one;
7-(1H-benzo[d]imidazol-4-yl)-1-(2-(tetrahydro-2H-pyran-4-yl)ethyl)-3,4-dihydropyrazino[2,3-b]pyrazin-2(1H)-one;
7-(1H-pyrrolo[2,3-b]pyridin-4-yl)-1-(2-(tetrahydro-2H-pyran-4-yl)ethyl)-3,4-dihydropyrazino[2,3-b]pyrazin-2(1H)-one;
7-(6-(1H-1,2,4-triazol-3-yl)pyridin-3-yl)-1-((*trans*-4-methoxycyclohexyl)methyl)-3,4-dihydropyrazino[2,3-b]pyrazin-2(1H)-one;
7-(6-(1H-1,2,4-triazol-3-yl)pyridin-3-yl)-1-((*trans*-4-hydroxycyclohexyl)methyl)-3,4-dihydropyrazino[2,3-b]pyrazin-2(1H)-one;
7-(6-(1H-1,2,4-triazol-3-yl)pyridin-3-yl)-1-(*cis*-4-hydroxycyclohexyl)-3,4-dihydropyrazino[2,3-b]pyrazin-2(1H)-one;
7-(5-fluoro-2-methyl-4-(1H-1,2,4-triazol-3-yl)phenyl)-1-(*cis*-4-hydroxycyclohexyl)-3,4-dihydropyrazino[2,3-b]pyrazin-2(1H)-one;
7-(6-(1H-1,2,4-triazol-3-yl)pyridin-3-yl)-1-(tetrahydro-2H-pyran-4-yl)-3,4-dihydropyrazino[2,3-b]pyrazin-2(1H)-one;
7-(6-(1H-1,2,4-triazol-3-yl)pyridin-3-yl)-1-(2-methoxyethyl)-3,4-dihydropyrazino(2,3-b]pyrazin-2(1H)-one;
7-(6-(1H-1,2,4-triazol-3-yl)pyridin-3-yl)-1-ethyl-3,4-dihydropyrazino[2,3-b]pyrazin-2(1H)-one;
7-(5-fluoro-2-methyl-4-(1H-1,2,4-triazol-3-yl)phenyl)-1-((*cis-*4-hydroxycyclohexyl)methyl)-3,4-dihydropyrazino[2,3-b]pyrazin-2(1H)-one;
7-(5-fluoro-2-methyl-4-(1H-1,2,4-triazol-3-yl)phenyl)-1-(tetrahydro-2H-pyran-4-yl)-3,4-dihydropyrazino[2,3-b]pyrazin-2(1H)-one;
7-(1H-indol-4-yl)-1-(2-(tetrahydro-2H-pyran-4-yl)ethyl)-3,4-dihydropyrazino[2,3-b]pyrazin-2(1H)-one;
7-(5-fluoro-2-methyl-4-(1H-1,2,4-triazol-3-yl)phenyl)-1-((*trans*-4-hydroxycyclohexyl)methyl)-3,4-dihydropyrazino[2,3-b]pyrazin-2(1H)-one;
7-(6-(1H-1,2,4-triazol-3-yl)pyridin-3-yl)-1-((*cis*-4-hydroxycyclohexyl)methyl)-3,4-dihydropyrazino[2,3-b]pyrazin-2(1H)-one;
7-(6-(1H-1,2,4-triazol-3-yl)pyridin-3-yl)-1-(*trans*-4-hydroxycyclohexyl)-3,4-dihydropyrazino[2,3-b]pyrazin-2(1H)-one;
7-(6-(1H-1,2,4-triazol-3-yl)pyridin-3-yl)-1-(*trans*-4-methoxycyclohexyl)-3,4-dihydropyrazino[2,3-b]pyrazin-2(1H)-one;
7-(6-(1H-1,2,4-triazol-3-yl)pyridin-3-yl)-1-isopropyl-3,4-dihydropyrazino[2,3-b]pyrazin-2(1H)-one;
7-(5-fluoro-2-methyl-4-(1H-1,2,4-triazol-3-yl)phenyl)-1-(*trans*-4-methoxycyclohexyl)-3,4-dihydropyrazino[2,3-b]pyrazin-2(1H)-one;
7-(5-fluoro-2-methyl-4-(1H-1,2,4-triazol-3-yl)phenyl)-1-(*trans*-4-hydroxycyclohexyl)-3,4-dihydropyrazino[2,3-b]pyrazin-2(1H)-one;
7-(5-fluoro-2-methyl-4-(1H-1,2,4-triazol-3-yl)phenyl)-1-(2-methoxyethyl)-3,4-dihydropyrazino[2,3-b]pyrazin-2(1H)-one;
7-(5-fluoro-2-methyl-4-(1H-1,2,4-triazol-3-yl)phenyl)-1-isopropyl-3,4-dihydropyrazino[2,3-b]pyrazin-2(1H)-one;
1-ethyl-7-(5-fluoro-2-methyl-4-(1H-1,2,4-triazol-3-yl)phenyl)-3,4-dihydropyrazino[2,3-b]pyrazin-2(1H)-one;
7-(2-hydroxypyridin-4-yl)-1-(2-(tetrahydro-2H-pyran-4-yl)ethyl)-3,4-dihydropyrazino[2,3-b]pyrazin-2(1H)-one;
1-isopropyl-7-(4-methyl-6-(1H-1,2,4-triazol-3-yl)pyridin-3-yl)-3,4-dihydropyrazino[2,3-b]pyrazin-2(1H)-one;
5-(8-isopropyl-7-oxo-5,6,7,8-tetrahydropyrazino[2,3-b]pyrazin-2-yl)-4-methylpicolinamide; 7-(1H-indazol-4-yl)-1-(2-(tetrahydro-2H-pyran-4-yl)ethyl)-3,4-dihydropyrazino[2,3-b]pyrazin-2(1H)-one;
7-(2-aminopyrimidin-5-yl)-1-(2-(tetrahydro-2H-pyran-4-yl)ethyl)-3,4-dihydropyrazino[2,3-b]pyrazin-2(1H)-one;
7-(2-aminopyridin-4-yl)-1-(2-(tetrahydro-2H-pyran-4-yl)ethyl)-3,4-dihydropyrazino[2,3-b]pyrazin-2(1H)-one;
7-(6-(methylamino)pyridin-3-yl)-1-(2-(tetrahydro-2H-pyran-4-yl)ethyl)-3,4-dihydropyrazino[2,3-b]pyrazin-2(1H)-one;
7-(6-hydroxypyridin-3-yl)-1-(2-(tetrahydro-2H-pyran-4-yl)ethyl)-3,4-dihydropyrazino[2,3-b]pyrazin-2(1H)-one;
7-(4-(1H-pyrazol-3-yl)phenyl)-1-(2-methoxyethyl)-3,4-dihydropyrazino[2,3-b]pyrazin-2(1H)-one;
7-(pyridin-3-yl)-1-(2-(tetrahydro-2H-pyran-4-yl)ethyl)-3,4-dihydropyrazino[2,3-b]pyrazin-2(1H)-one;
7-(1H-indazol-4-yl)-1-(2-methoxyethyl)-3,4-dihydropyrazino[2,3-b]pyrazin-2(1H)-one;
7-(1H-indazol-6-yl)-1-(2-methoxyethyl)-3,4-dihydropyrazino[2,3-b]pyrazin-2(1H)-one;
7-(pyrimidin-5-yl)-1-(2-(tetrahydro-2H-pyran-4-yl)ethyl)-3,4-dihydropyrazino[2,3-b]pyrazin-2(1H)-one;
7-(6-methoxypyridin-3-yl)-1-(2-(tetrahydro-2H-pyran-4-yl)ethyl)-3,4-dihydropyrazino[2,3-b]pyrazin-2(1H)-one;
1-(2-methoxyethyl)-7-(1H-pyrrolo[2,3-b]pyridin-5-yl)-3,4-dihydropyrazino[2,3-b]pyrazin-2(1H)-one;
1-ethyl-7-(1H-pyrrolo[2,3-b]pyridin-5-yl)-3,4-dihydropyrazino[2,3-b)pyrazin-2(1H)-one; 1-ethyl-7-(1H-indazol-4-yl)-3,4-dihydropyrazino[2,3-b]pyrazin-2(1H)-one;
7-(pyridin-4-yl)-1-(2-(tetrahydro-2H-pyran-4-yl)ethyl)-3,4-dihydropyrazino[2,3-b]pyrazin-2(1H)-one;
7-(6-aminopyridin-3-yl)-1-(2-(tetrahydro-2H-pyran-4-yl)ethyl)-3,4-dihydropyrazino[2,3-b)pyrazin-2(1H)-one;
1-methyl-7-(2-methyl-6-(4H-1,2,4-triazol-3-yl)pyridin-3-yl)-3,4-dihydropyrazino[2,3-b]pyrazin-2(1H)-one;
2-(2-hydroxypropan-2-yl)-5-(8-(*trans*-4-methoxycyclohexyl)-7-oxo-5,6,7,8-tetrahydropyrazino[2,3-b]pyrazin-2-yl)pyridine 1-oxide;
4-methyl-5-(7-oxo-8-((tetrahydro-2H-pyran-4-yl)methyl)-5,6,7,8-tetrahydropyrazino[2,3-b]pyrazin-2-yl)picolinamide;
5-(8-((*cis*-4-methoxycyclohexyl)methyl)-7-oxo-5,6,7,8-tetrahydropyrazino[2,3-b]pyrazin-2-yl)-4-methylpicolinamide;
7-(1H-pyrazol-4-yl)-1-(2-(tetrahydro-2H-pyran-4-yl)ethyl)-3,4-dihydropyrazino[2,3-b]pyrazin-2(1H)-one;
2-(*trans*-4-methoxycyclohexyl)-7-(4-methyl-6-(1H-1,2,4-triazol-3-yl)pyridin-3-yl)-3,4-dihydropyrazino[2,3-b]pyrazin-2(1H)-one;
3-((7-(2-methyl-6-(4H-1,2,4-triazol-3-yl)pyridin-3-yl)-2-oxo-3,4-dihydropyrazino[2,3-b]pyrazin-1(2H)-yl)methyl)benzonitrile;
1-((*trans*-4-methoxycyclohexyl)methyl)-7-(4-methyl-6-(1H-1,2,4-triazol-3-yl)pyridin-3-yl)-3,4-dihydropyrazino[2,3-b]pyrazin-2(1H)-one;
3-(7-oxo-8-(2-(tetrahydro-2H-pyran-4-yl)ethyl)-5,6,7,8-tetrahydropyrazino[2,3-b]pyrazin-2-yl)benzamide;
5-(8-((*trans*-4-methoxycyclohexyl)methyl)-7-oxo-5,6,7,8-tetrahydropyrazino[2,3-b]pyrazin-2-yl)-4-methylpicolinamide;
3-((7-(6-(2-hydroxypropan-2-yl)pyridin-3-yl)-2-oxo-3,4-dihydropyrazino[2,3-b]pyrazin-1(2H)-yl)methyl)benzonitrile;
7-(6-(2-hydroxypropan-2-yl)pyridin-3-yl)-1-((1R,3R)-3-methoxycyclopentyl)-3,4-dihydropyrazino[2,3-b]pyrazin-2(1H)-one;
7-(6-(2-hydroxypropan-2-yl)pyridin-3-yl)-1-((1S,3R)-3-methoxycyclopentyl)-3,4-dihydropyrazino[2,3-b]pyrazin-2(1H)-one;
7-(6-(2-hydroxypropan-2-yl)pyridin-3-yl)-1-((1S,3S)-3-methoxycyclopentyl)-3,4-dihydropyrazino[2,3-b]pyrazin-2(1H)-one;
7-(6-(2-hydroxypropan-2-yl)pyridin-3-yl)-1-((1R,3S)-3-methoxycyclopentyl)-3,4-dihydropyrazino[2,3-b]pyrazin-2(1H)-one;
7-(1H-indazol-6-yl)-1-(2-(tetrahydro-2H-pyran-4-yl)ethyl)-3,4-dihydropyrazino[2,3-b]pyrazin-2(1H)-one;
7-(2-methyl-6-(4H-1,2,4-triazol-3-yl)pyridin-3-yl)-1-(2-morpholinoethyl)-3,4-dihydropyrazino[2,3-b]pyrazin-2(1H)-one;
1-(*trans*-4-hydroxycyclohexyl)-7-(2-methyl-6-(4H-1,2,4-triazol-3-yl)pyridin-3-yl)-3,4-dihydropyrazino[2,3-b]pyrazin-2(1H)-one;
1-(*cis*-4-hydroxycyclohexyl)-7-(2-methyl-6-(4H-1,2,4-triazol-3-yl)pyridin-3-yl)-3,4-dihydropyrazino[2,3-b]pyrazin-2(1H)-one;
7-(6-(2-hydroxypropan-2-yl)pyridin-3-yl)-1-(2-morpholinoethyl)-3,4-dihydropyrazino[2,3-b]pyrazin-2(1H)-one;
1-isopropyl-7-(2-methyl-6-(4H-1,2,4-triazol-3-yl)pyridin-3-yl)-3,4-dihydropyrazino[2,3-b]pyrazin-2(1H)-one;
7-(1H-imidazo[4,5-b]pyridin-6-yl)-1-(2-(tetrahydro-2H-pyran-4-yl)ethyl)-3,4-dihydropyrazino[2,3-b]pyrazin-2(1H)-one;
1-((*cis*-4-methoxycyclohexyl)methyl)-7-(2-methyl-6-(1H-1,2,4-triazol-3-yl)pyridin-3-yl)-3,4-dihydropyrazino[2,3-b]pyrazin-2(1H)-one;
1-(*trans*-4-hydroxycyclohexyl)-7-(6-(2-hydroxypropan-2-yl)pyridin-3-yl)-3,4-dihydropyrazino[2,3-b]pyrazin-2(1H)-one:
1-(*cis*-4-hydroxycyclohexyl)-7-(6-(2-hydroxypropan-2-yl)pyridin-3-yl)-3,4-dihydropyrazino[2,3-b]pyrazin-2(1H)-one;
4-(7-oxo-8-(2-(tetrahydro-2H-pyran-4-yl)ethyl)-5,6,7,8-tetrahydropyrazino[2,3-b]pyrazin-2-yl)benzamide;
7-(1H-indazol-5-yl)-1-(2-(tetrahydro-2H-pyran-4-yl)ethyl)-3,4-dihydropyrazino[2,3-b]pyrazin-2(1H)-one;
7-(1H-pyrrolo[2,3-b]pyridin-5-yl)-1-(2-(tetrahydro-2H-pyran-4-yl)ethyl)-3,4-dihydropyrazino[2,3-b]pyrazin-2(1H)-one;
7-(2-methyl-6-(4H-1,2,4-triazol-3-yl)pyridin-3-yl)-1-(tetrahydro-2H-pyran-4-yl)-3,4-dihydropyrazino[2,3-b]pyrazin-2(1H)-one;
1-((1S,3R)-3-methoxycyclopentyl)-7-(2-methyl-6-(4H-1,2,4-triazol-3-yl)pyridin-3-yl)-3,4-dihydropyrazino[2,3-b]pyrazin-2(1H)-one;
1-((1R,3R)-3-methoxycyclopentyl)-7-(2-methyl-6-(4H-1,2,4-triazol-3-yl)pyridin-3-yl)-3,4-dihydropyrazino[2,3-b]pyrazin-2(1H)-one;
1-((1R,3S)-3-methoxycyclopentyl)-7-(2-methyl-6-(4H-1,2,4-triazol-3-yl)pyridin-3-yl)-3,4-dihydropyrazino[2,3-b]pyrazin-2(1H)-one;
1-((1S,3S)-3-methoxycyclopentyl)-7-(2-methyl-6-(4H-1,2,4-triazol-3-yl)pyridin-3-yl)-3,4-dihydropyrazino[2,3-b]pyrazin-2(1H)-one;
7-(1H-indol-5-yl)-1-(2-(tetrahydro-2H-pyran-4-yl)ethyl)-3,4-dihydropyrazino[2,3-b]pyrazin-2(1H)-one;
1-ethyl-7-(2-methyl-6-(4H-1,2,4-triazol-3-yl)pyridin-3-yl)-3,4-dihydropyrazino[2,3-b]pyrazin-2(1H)-one;
7-(1H-indol-6-yl)-1-(2-(tetrahydro-2H-pyran-4-yl)ethyl)-3,4-dihydropyrazino[2,3-b]pyrazin-2(1H)-one;
7-(4-(2-hydroxypropan-2-yl)phenyl)-1-(*trans*-4-methoxycyclohexyl)-3,4-dihydropyrazino[2,3-b]pyrazin-2(1H)-one;
7-(6-(2-hydroxypropan-2-yl)pyridin-3-yl)-1-(tetrahydro-2H-pyran-4-yl)-3,4-dihydropyrazino[2,3-b]pyrazin-2(1H)-one;
1-((*trans*-4-methoxycyclohexyl)methyl)-7-(2-methyl-6-(1H-1,2,4-triazol-3-yl)pyridin-3-yl)-3,4-dihydropyrazino[2,3-b]pyrazin-2(1H)-one;
7-(6-(2-hydroxypropan-2-yl)pyridin-3-yl)-1-((*cis*-4-methoxycyclohexyl)methyl)-3,4-dihydropyrazino[2,3-b]pyrazin-2(1H)-one;
1-(2-methoxyethyl)-7-(4-methyl-2-(methylamino)-1H-benzo[d]imidazol-6-yl)-3,4-dihydropyrazino[2,3-b]pyrazin-2(1H)-one;
7-(7-methyl-2-oxo-2,3-dihydro-1H-benzo[d]imidazol-5-yl)-1-((tetrahydro-2H-pyran-4-yl)methyl)-3,4-dihydropyrazino[2,3-b]pyrazin-2(1H)-one;
7-(2-methyl-4-(4H-1,2,4-triazol-3-yl)phenyl)-3,4-dihydropyrazino[2,3-b]pyrazin-2(1H)-one; 1-(2-methoxyethyl)-7-(4-methyl-6-(1H-1,2,4-triazol-3-yl)pyridin-3-yl)-3,4-dihydropyrazino[2,3-b]pyrazin-2(1H)-one;
1-benzyl-7-(2-methyl-4-(4H-1,2,4-triazol-3-yl)phenyl)-3,4-dihydropyrazino[2,3-b]pyrazin-2(1-H)-one;
7-(3-fluoro-4-(4H-1,2,4-triazol-3-yl)phenyl)-1-(2-methoxyethyl)-3,4-dihydropyrazino[2,3-b]pyrazin-2(1H)-one;
7-(3-fluoro-4-(4H-1,2,4-triazol-3-yl)phenyl)-1-(2-(tetrahydro-2H-pyran-4-yl)ethyl)-3,4-dihydropyrazino[2,3-b]pyrazin-2(1H)-one;
7-(3-fluoro-2-methyl-4-(1H-1,2,4-triazol-3-yl)phenyl)-1-(2-methoxyethyl)-3,4-dihydropyrazino[2,3-b]pyrazin-2(1H)-one;
1-(*trans*-4-methoxycyclohexyl)-7-(2-methyl-6-(4H-1,2,4-triazol-3-yl)pyridin-3-yl)-3,4-dihydropyrazino[2,3-b]pyrazin-2(1H)-one;
7-(5-fluoro-2-methyl-4-(4H-1,2,4-triazol-3-yl)phenyl)-1-(2-(tetrahydro-2H-pyran-4-yl)ethyl)-3,4-dihydropyrazino[2,3-b]pyrazin-2(1H)-one;
7-(3-fluoro-2-methyl-4-(1H-1,2,4-triazol-3-yl)phenyl)-1-(2-(tetrahydro-2H-pyran-4-yl)ethyl)-3,4-dihydropyrazino[2,3-b]pyrazin-2(1H)-one;
1-(2-methoxyethyl)-7-(2-methyl-6-(4H-1,2,4-triazol-3-yl)pyridin-3-yl)-3,4-dihydropyrazino[2,3-b]pyrazin-2(1H)-one;
7-(6-(2-hydroxypropan-2-yl)pyridin-3-yl)-1-((*trans*-4-methoxycyclohexyl)methyl)-3,4-dihydropyrazino[2,3-b]pyrazin-2(1H)-one;
1-(cyclopentylmethyl)-7-(6-(2-hydroxypropan-2-yl)pyridin-3-yl)-3,4-dihydropyrazino[2,3-b]pyrazin-2(1H)-one;
7-(4-(2-hydroxypropan-2-yl)phenyl)-1-(2-methoxyethyl)-3,4-dihydropyrazino[2,3-b]pyrazin-2(1H)-one;
(S)-7-(6-(1-hydroxyethyl)pyridin-3-yl)-1-(2-(tetrahydro-2H-pyran-4-yl)ethyl)-3,4-dihydropyrazino[2,3-b]pyrazin-2(1H)-one;
(R)-7-(6-(1-hydroxyethyl)pyridin-3-yl)-1-(2-(tetrahydro-2H-pyran-4-yl)ethyl)-3,4-dihydropyrazino[2,3-b]pyrazin-2(1H)-one;
7-(2-methyl-6-(4H-1,2,4-triazol-3-yl)pyridin-3-yl)-1-((tetrahydro-2H-pyran-4-yl)methyl)-3,4-dihydropyrazino[2,3-b]pyrazin-2(1H)-one;
7-(4-(2-hydroxypropan-2-yl)phenyl)-1-(2-(tetrahydro-2H-pyran-4-yl)ethyl)-3,4-dihydropyrazino[2,3-b]pyrazin-2(1H)-one;
7-(6-(2-hydroxypropan-2-yl)pyridin-3-yl)-1-(4-(trifluoromethyl)benzyl)-3,4-dihydropyrazino[2,3-b]pyrazin-2(1H)-one;
7-(6-(2-hydroxypropan-2-yl)pyridin-3-yl)-1-(3-(trifluoromethyl)benzyl)-3,4-dihydropyrazino[2,3-b]pyrazin-2(1H)-one;
7-(6-(2-hydroxypropan-2-yl)pyridin-3-yl)-1-(3-methoxypropyl)-3,4-dihydropyrazino[2,3-b]pyrazin-2(1H)-one;
7-(4-methyl-6-(1H-1,2,4-triazol-3-yl)pyridin-3-yl)-1-(2-(tetrahydro-2H-pyran-4-yl)ethyl)-3,4-dihydropyrazino[2,3-b]pyrazin-2(1H)-one;
7-(6-(2-hydroxypropan-2-yl)pyridin-3-yl)-1-(2-methoxyethyl)-3,4-dihydropyrazino[2,3-b]pyrazin-2(1H)-one;
7-(6-(2-hydroxypropan-2-yl)pyridin-3-yl)-1-((tetrahydro-2H-pyran-4-yl)methyl)-3,4-dihydropyrazino[2,3-b]pyrazin-2(1H)-one;
7-(4-methyl-2-(methylamino)-1H-benzo[d]imidazol-6-yl)-1-((tetrahydro-2H-pyran-4-yl)methyl)-3,4-dihydropyrazino[2,3-b]pyrazin-2(1H)-one;
7-(2-amino-4-methyl-1H-benzo[d]imidazol-6-yl)-1-((tetrahydro-2H-pyran-4-yl)methyl)-3,4-dihydropyrazino[2,3-b]pyrazin-2(1H)-one;
7-(2-methyl-6-(4H-1,2,4-triazol-3-yl)pyridin-3-yl)-1-(2-(tetrahydro-2H-pyran-4-yl)ethyl)-3,4-dihydropyrazino[2,3-b]pyrazin-2(1H)-one;
(R)-7-(6-(2-hydroxypropan-2-yl)pyridin-3-yl)-3-methyl-1-(2-(tetrahydro-2H-pyran-4-yl)ethyl)-3,4-dihydropyrazino[2,3-b]pyrazin-2(1H)-one;
(S)-7-(6-(2-hydroxypropan-2-yl)pyridin-3-yl)-3-methyl-1-(2-(tetrahydro-2H-pyran-4-yl)ethyl)-3,4-dihydropyrazino[2,3-b]pyrazin-2(1H)-one;
7-(6-(2-hydroxypropan-2-yl)pyridin-3-yl)-3,3-dimethyl-1-(2-(tetrahydro-2H-pyran-4-yl)ethyl)-3,4-dihydropyrazino[2,3-b]pyrazin-2(1H)-one;
7-(2-amino-4-methyl-1H-benzo[d]imidazol-6-yl)-1-(2-(tetrahydro-2H-pyran-4-yl)ethyl)-3,4-dihydropyrazino[2,3-b]pyrazin-2(1H)-one:
7-(6-(2-hydroxypropan-2-yl)pyridin-3-yl)-1-(2-(tetrahydro-2H-pyran-4-yl)ethyl)-3,4-dihydropyrazino[2,3-b]pyrazin-2(1H)-one;
7-(2-methyl-4-(1H-1,2,4-triazol-3-yl)phenyl)-1-(2-(tetrahydro-2H-pyran-4-yl)ethyl)-3,4-dihydropyrazino[2,3-b]pyrazin-2(1H)-one;
7-(4-(1H-1,2,4-triazol-5-yl)phenyl)-1-(2-(tetrahydro-2H-pyran-4-yl)ethyl)-3,4-dihydropyrazino[2,3-b]pyrazin-2(1H)-one;
1-(1-hydroxypropan-2-yl)-7-(2-methyl-6-(1H-1,2,4-triazol-3-yl)pyridin-3-yl)-3,4-dihydropyrazino[2,3-b]pyrazin-2(1H)-one;
1-(2-hydroxyethyl)-7-(2-methyl-6-(1H-1,2,4-triazol-3-yl)pyridin-3-yl)-3,4-dihydropyrazino[2,3-b]pyrazin-2(1H)-one; or a pharmaceutically acceptable salt thereof. [According to the present invention, the TOR kinase inhibitor is 7-(6-(2-hydroxypropan-2-yl)pyridin-3-yl)-1-(trans-4-methoxycyclohexyl)-3,4-dihydropyrazino[2,3-b]pyrazin-2(1H)-one].

### 6. EXAMPLES

### 6.1 FLOW CYTOMETRY ASSAY

### Antibodies

The following antibodies were used: (i) Phospho-4EBP1 (T37/46), Alexa Fluor 647 Conjugate (Cell Signaling, cat# 5123); (ii) Mouse Anti-human CD3 (FITC)(BD Biosciences, cat# 555332); and (iii) Mouse Anti-human CD91 (PE) (BD Biosciences, cat# 550497).

### Reagents

The following reagents were used: (i) Phospho-4EBP1 blocking peptide (100µg @ 1 mg/ml), (Cell Signaling Technology, cat#1052) (store at -20 °C); (ii) Phospho-4EBP1 blocking peptide buffer, containing of 20 mM potassium phosphate (pH 7.0), 50 mM NaCl, 0.1 mM EDTA, 1 mg/ml BSA and 5% glycerol (store at -20 °C); (iii) BD Stain Buffer (BSA), Cat# 554657, BD Biosciences, containing Dulbeccos Phosphate Buffered Saline (DPBS) pH 7.4, 0.2% (w/v) bovine serum albumin (BSA) and 0.09% sodium azide (NaN₃) (store at 4 °C); (iv) BD PhosFlow Lyse/fix buffer (5x), Cat# 558049, BD Biosciences, a buffered solution containing <40% formaldehyde and <50% diethylene glycol (store at room temperature); (v) BD PhospFlow Perm Buffer III, cat# 558050, BD Biosciences, a buffered solution containing 90% methanol (store at room temperature); (vi) BD Stabilizing Fixative (3x), cat# 338036, BD Biosciences (store at room temperature); (vii) 96 well

Microtest U-Bottom plates; cat# 353077, Falcon; (viii) BD vacutainer tubes (heparin), cat# 367874 (plastic), cat# 366480 (glass); (ix) Compound 1, [wherein Compound 1 is 7-(6-(2-hydroxypropan-2-yl)pyridin-3-yl)-1-(trans-4-methoxycyclohexyl)-3,4 dihydropyrazino[2,3-b]pyrazin-2(1H)-one] an illustrative TOR kinase inhibitor found in International Patent Publication No. WO 2010/062571, published on June 3, 2010, the contents of which are incorporated herein by reference in their entirety (*see, e.g.,* Section 4.2, pages 15-43 and Table 1); (x) normal healthy volunteer whole blood obtained from TSRI Normal Blood Donor Service, La Jolla, CA; and (xi) patient whole blood obtained from Conversant Biologics.

### Treatment of Whole Blood

Whole blood was collected from healthy donors/patients into vacutainer tubes containing sodium heparin (BD, cat# 367874 (plastic), cat# 366480 (glass)). Care was taken to mix the blood thoroughly in the tube to prevent clotting.

Compound dilutions were prepared as follows: Compound 1 was dissolved in 100% DMSO to make 30 mM or 10 mM stock concentration, the 30 mM or 10 mM stock concentrations of Compound 1 were diluted in 100% DMSO to make 5 mM, 0.5 mM and 0.1 mM stock concentrations and 1:100 dilutions of the 5 mM, 0.5 mM and 0.1 mM stock concentrations were made into media, with the final concentrations of Compound 1 in media being 50 µM, 5 µM and 1 µM, respectively. Dilutions were kept at room temperature before addition to the blood.

1.8 mL of whole blood was transferred to a 50 mL conical tube and treated with 200 µL of Compound 1 diluted in media for 2 hours in the dark at room temperature. The final Compound 1 concentration was 5 µM, 0.5 µM and 0.1 µM, respectively. The final DMSO concentration was be 0.1%. Each treatment was done in triplicate.

### Fixing and Permeabilization

While waiting for blood treatments to finish, 5x BD Lyse/Fix Buffer (cat# 558049) was diluted with distilled (or deionized) water. The 1x Lyse/Fix buffer was prewarmed in a 37 °C water bath for 5-10 minutes before use.

Cells were lysed/fixed immediately by mixing 1 volume of blood with 20 volumes of 1x Lyse/fix buffer (for the 2 mL of blood + Compound 1, 40 mL of 1x Lyse/fix buffer was added) and mixed thoroughly by inverting the tube several times.

The Lyse/fix and blood mixture was incubated in a 37 °C water bath for 10 minutes.

Cells were pelleted by centrifugation at 800x g for 5 minutes and supernatant was removed by aspiration.

Cells were suspended with 1.3 mL of cold PBS and transferred to 1.5 ml eppendoff tube. Cells were pelleted by centrifugation at 800x g for 5 minutes and supernatant was removed by aspiration.

Cells were washed with 1 mL of cold PBS, spun at 800x g for 5 minutes and supernatant was removed by aspiration. (Cells can also be frozen at -80 °C directly at this step for later usage. If desired, cells from the 1.8 ml of blood can be split into 2 vials for 2 sets of staining reactions). For cells frozen at -80 °C, frozen tubes with cells are transferred onto ice and cells are suspended with 1 mL of cold PBS. Cells are pelleted by centrifugation at 800x g for 5 minutes and supernatant is removed by aspiration.

Cells were suspended and permeabilized by adding 1 mL of Perm Buffer III (1-10x10⁶ cells) and incubated on ice for 15 minutes (The cell pellet should be well resuspended without the presence of chunks. It is important that cells are treated for no more than 30 minutes. Over or under permeabilization of the cells can affect the overall phospho-epitope signals).

Cells were pelleted by centrifugation at 880x g for 5 minutes and supernatant was removed by aspiration.

Cells were washed with 1 mL of staining buffer, centrifuged at 880x g for 5 minutes and supernatant was removed by aspiration.

### Antibody Addition and Data Collection

Cells were batch stained with surface antibodies, making sure that surface markers were compatible with Perm Buffer III system (if antibody is not compatible, surface labeling with antibody before treating cells with Perm Buffer III should be tried. Becton Dickinson recommends using 20 µl of each surface antibody for every 100 µl reaction).

For PBMC cells from 1.8 mL whole blood, the cell pellet from above was resuspended in 30 µL of staining buffer (40 µL of staining buffer is used if only staining CD91 cells). 10 µL of anti-CD3 and 10 µL of anti-CD91 were added to cells, mixed thoroughly and incubated at room temperature for 30 minutes in the dark (PC3 cells do not need to surface staining).

While waiting for the surface staining, 96 well U-bottom plates with phospho-antibodies and blocking peptide or buffer were prepared. p4EBP1 staining solution, blocking peptide solution and control solution were also prepared during the waiting period, as follows: p4EBP1 staining solution: 10 parts peptide buffer + 2 parts anti-p4EBP1 antibody: blocking peptide solution: 10 parts blocking peptide + 2 parts anti-p4EBP1 antibody: and control solution: 10 parts peptide buffer + 2 parts staining buffer.

12 µL of staining solution, blocking peptide solution or control solution were added according to the following plate setting:

| | **1** | **2** | **3** | **4** | **5** | |
|---|---|---|---|---|---|---|
| **A** | DMSO-treat 1 | 5µM-treat 1 | 0.5µM-treat 1 | 0.1µM-treat 1 | | p4EBP1 staining |
| **B** | DMSO-treat 2 | 5µM-treat 2 | 0.5µM-treat 2 | 0.1µM-treat 2 | PC3 DMSO | |
| **C** | DMSO-treat 3 | 5µM-treat 3 | 0.5µM-treat 3 | 0.1µM-treat 3 | PC3-5µM | |
| **D** | DMSO-treat 1 | DMSO-treat 2 | DMSO-treat 3 | PC3 DMSO | PC3-5µM | p4EBP1-peptide |
| **E** | DMSO-treat 1 | DMSO-treat 2 | DMSO-treat 3 | PC3 DMSO | PC3-5µM) | control |

88 µL of diluted cells were transferred to each well following the above plate layout. The plate was gently shaken to mix staining buffer with cells.

The plate was shaken at 25 rpm in the dark at room temperature for 30 minutes. Cells were pelleted in the plate by centrifugation at 880x g for 5 minutes. The supernatant was removed quickly by inverting the plate and expelling the contents out.

The plate was shaken to loosen the cell pellet in the 96-well plate. Cells were washed with 200 µl of staining buffer per well. Cells were pelleted in the plate by centrifugation at 880x g for 5 minutes. Supernatant was removed by quickly inverting the plate and expelling contents out.

The plate was again shaken at 25 rpm in the dark at room temperature for 30 minutes. Cells were pelleted in the plate by centrifugation at 880x g for 5 minutes. The supernatant was removed quickly by inverting the plate and expelling the contents out.

During the centrifugation. the 3x Concentrate BD Stabilizing Fixative was diluted 1:3 with deionized water at room temperature.

The plate was shaken to loosen the cell pellet. Cells were resuspended in 200 µl of 1x BD Stabilizing Fixative.

The plate was read on a FACS Calibur flow cytometer equipped with a 96 well plate reader. Plates that cannot be read right away should be covered in foil to protect from light and stored at 4 °C. Plates should be read within 2-3 hours.

Data analysis was be carried out using FlowJo (flow cytometry analysis software) from Tree Star, Inc. Mean/median fluorescence intensity and percent inhibition of protein phosphorylation was assessed.

Results are illustrated in FIGS. 1-4.

### 6.2 mTOR HTR-FRET ASSAY

The following is an example of an assay that can be used to determine the mTOR inhibitory activity of a test compound. Test compounds are dissolved in DMSO and prepared as 10 mM stocks and diluted appropriately for the experiments. Reagents are prepared as follows:

"Simple TOR buffer" (used to dilute high glycerol TOR fraction): 10 mM Tris pH 7.4, 100 mM NaCl, 0.1% Tween-20, 1 mM DTT. Invitrogen mTOR (cat#PR8683A) is diluted in this buffer to an assay concentration of 0.200 µg/mL.

ATP/Substrate solution: 0.075 mM ATP, 12.5 mM MnCl₂, 50 mM Hepes, pH 7.4, 50 mM β-GOP, 250 nM Microcystin LR, 0.25 mM EDTA, 5 mM DTT, and 3.5 µg/mL GST-p70S6.

Detection reagent solution: 50 mM HEPES, pH 7.4, 0.01% Triton X-100, 0.01% BSA, 0.1 mM EDTA, 12.7 µg/mL Cy5-αGST Amersham (Cat#PA92002V), 9 ng/mL α-phospho p70S6 (Thr389) (Cell Signaling Mouse Monoclonal #9206L). 627 ng/mL α-mouse Lance Eu (Perkin Elmer Cat#AD0077).

To 20 µL of the Simple mTor buffer is added 0.5 µL of test compound in DMSO. To initiate the reaction 5 µL of ATP/Substrate solution is added to 20 µL of the Simple TOR buffer solution (control) and to the compound solution prepared above. The assay is stopped after 60 minutes by adding 5 µL of a 60 mM EDTA solution: 10 µL of detection reagent solution is then added and the mixture is allowed to sit for at least 2 hours before reading on a Perkin-Elmer Envision Microplate Reader set to detect LANCE Eu TR-FRET (excitation at 320 nm and emission at 495/520 nm).

### 6.3 CLINICAL STUDY

The patient population was comprised of men and women, over 18 years old.

Compound 1 was administered orally, in an uninterrupted once-daily schedule. Each dose was taken in the morning, with the patient having fasted overnight (minimum of 6 hrs).

Each patient was administered a single dose of Compound 1 (Day -1). followed by a 24-hour wash out period and a 48-hour observation and pharmacokinetic sample collection period, which was then followed on Day 1 by daily dosing for 28 days.

The following initial doses were administered: Cohort 1 = 7.5 mg; Cohort 2 = 15 mg; Cohort 3 = 30 mg; Cohort 4 = 45 mg. Initial cohorts of one subject were given Compound 1 in dose increments of 100% (i.e., doubling the dose level each time) until the first instance of first-course grade 2 or higher toxicity suspected to be Compound 1 related.

Results are illustrated in FIGS. 5 and 6.

## Claims

1. An in vitro method for detecting or measuring the inhibition of TOR kinase activity in a subject, comprising the use of flow cytometry to measure the amount of phosphorylated 4EBP1 in biological samples which have been obtained from said subject prior to and after the administration of a TOR kinase inhibitor, wherein the TOR kinase inhibitor is 7-(6-(2-hydroxypropan-2-yl)pyridin-3-yl)-1-(*trans*-4-methoxycyclohexyl)-3,4-dihydropyrazino[2,3-b]pyrazin-2(1H)-one or a pharmaceutically acceptable salt, clathrate, solvate, stereoisomer, tautomer, or prodrug thereof, wherein a decrease in the amount of the phosphorylated 4EBP1 confirms inhibition of TOR kinase activity in the subject.

2. The in vitro method of claim 1, which is a method for determining a dose-response relationship for the administration of a TOR kinase inhibitor to said subject, wherein said subject has been administered varying doses of said TOR kinase inhibitor and the amount of TOR kinase activity inhibition in said subject resulting from each dose of said TOR kinase inhibitor is determined by the use of flow cytometry to measure the amount of phosphorylated 4EBP1 in biological samples which have been obtained from said subject prior to and after each administration of said TOR kinase inhibitor.

3. The in vitro method of claim 1, which is a method for determining whether said subject is sensitive to a TOR kinase inhibitor, wherein said subject has been administered said TOR kinase inhibitor and it is determined whether or not said TOR kinase is inhibited in said subject by the use of flow cytometry to measure the amount of phosphorylated 4EBP1 in biological samples which have been obtained from said subject prior to and after the administration of said TOR kinase inhibitor.

4. The in vitro method of claim 1, which is a method for determining the effective amount of a TOR kinase inhibitor for the treatment or management of a disease in a patient, wherein said patient has been administered varying doses of said TOR kinase inhibitor and the amount of TOR kinase activity inhibition in said patient resulting from each dose of said TOR kinase inhibitor is determined by the use of flow cytometry to measure the amount of phosphorylated 4EBP1 in biological samples which have been obtained from said patient prior to and after each administration of said TOR kinase inhibitor.

5. A kit comprising one or more containers filled with an anti-p4EBP1 antibody, wherein the kit is used for measuring the amount of 4EBP1 in biological samples using flow cytometry, wherein the kit comprises a TOR kinase inhibitor, wherein the TOR kinase inhibitor is 7-(6-(2-hydroxypropan-2-yl)pyridin-3-yl)-1-(*trans*-4-methoxycyclohexyl)-3,4-dihydropyrazino[2,3-b]pyrazin-2(1H)-one or a pharmaceutically acceptable salt, clathrate, solvate, stereoisomer, tautomer, or prodrug thereof.

## Patentansprüche

1. In vitro Verfahren zur Bestimmung oder zum Messen der Inhibierung von TOR Kinaseaktivität bei einem Subjekt, umfassend die Verwendung von Durchflußzytometrie, um die Menge an phosphoryliertem 4EBP1 in biologischen Proben, die vor und nach der Verabreichung eines TOR Kinase-Inhibitors von dem Subjekt erhalten wurden, zu messen, wobei der TOR Kinase-Inhibitor 7-(6-(2-Hydroxypropan-2-yl)pyridin-3-yl)-1-(*trans*-4-methoxycyclohexyl)-3,4-dihydropyrazino[2,3-b]pyrazin-2(1H)-on oder ein pharmazeutisch annehmbares Salz, Clathrat, Solvat, Stereoisomer, Tautomer oder Prodrug davon ist, wobei eine Verringerung der Menge des phosphorylierten 4EBP1 die Inhibierung der TOR Kinaseaktivität in dem Subjekt bestätigt.

2. In vitro Verfahren nach Anspruch 1, das ein Verfahren zur Bestimmung einer Dosis-Antwort-Beziehung für die Verabreichung eines TOR Kinase-Inhibitors an das Subjekt ist, wobei dem Subjekt variierende Dosen des TOR Kinase-Inhibitors verabreicht wurden und die Menge der TOR Kinaseaktivitäts-Inhibierung in dem Subjekt, die von jeder Dosis des TOR Kinase-Inhibitors resultiert, durch die Verwendung von Durchflußzytometrie bestimmt wird, um die Menge an phosphoryliertem 4EBP1 in biologischen Proben, die vor und nach jeder Verabreichung des TOR Kinase-Inhibitors von dem Subjekt erhalten wurden, zu messen.

3. In vitro Verfahren nach Anspruch 1, das ein Verfahren zur Bestimmung ist, ob das Subjekt gegenüber einem TOR Kinase-Inhibitor empfindlich ist, wobei dem Subjekt der TOR Kinase-Inhibitor verabreicht wurde und es durch die Verwendung von Durchflußzytometrie bestimmt wird, ob die TOR Kinase in dem Subjekt inhibiert ist oder nicht, um die Menge an phosphoryliertem 4EBP1 in biologischen Proben, die vor und nach der Verabreichung des TOR Kinase-Inhibitors von dem Subjekt erhalten wurden, zu messen.

4. In vitro Verfahren nach Anspruch 1, das ein Verfahren zur Bestimmung der wirksamen Menge eines TOR Kinase-Inhibitors für die Behandlung oder das Managen einer Erkrankung bei einem Patienten ist, wobei dem Patient variierende Dosen des TOR Kinase-Inhibitors verabreicht wurden und die Menge der TOR Kinaseaktivitäts-Inhibierung bei dem Patient, die von jeder Dosis des TOR Kinase-Inhibitors resultiert, durch die Verwendung von Durchflußzytometrie bestimmt wird, um die Menge an phosphoryliertem 4EBP1 in biologischen Proben, die vor und nach jeder Verabreichung des TOR Kinase-Inhibitors von dem Patienten erhalten wurden, zu messen.

5. Kit umfassend einen oder mehrere Behälter, die mit einem anti-p4EBP1 Antikörper gefüllt sind, wobei das Kit für die Messung der Menge an 4EBP1 in biologischen Proben unter Verwendung von Durchflußzytometrie verwendet wird, wobei das Kit einen TOR Kinase-Inhibitor umfasst, wobei der TOR Kinase-Inhibitor 7-(6-(2-Hydroxypropan-2-yl)pyridin-3-yl)-1-(*trans*-4-methoxycyclohexyl)-3,4-dihydropyrazino[2,3-b]pyrazin-2(1H)-on oder ein pharmazeutisch annehmbares Salz, Clathrat, Solvat, Stereoisomer, Tautomer oder Prodrug davon ist.

## Revendications

1. Procédé in vitro pour détecter ou mesurer l'inhibition de l'activité d'une kinase TOR chez un sujet, comprenant l'utilisation de la cytométrie en flux pour mesurer la quantité de protéine 4EBP1 phosphorylée présente dans des échantillons biologiques qui ont été obtenus auprès dudit sujet avant et après l'administration d'un inhibiteur de kinase TOR, l'inhibiteur de kinase TOR étant la 7-(6-(2-hydroxypropan-2-yl)pyridin-3-yl)-1-(t*rans*-4-méthoxycyclohexyl)-3,4-dihydropyrazino[2,3-b]pyrazin-2(1H)-one ou un sel pharmaceutiquement acceptable, clathrate, solvate, stéréoisomère, tautomère, ou promédicament de celle-ci, dans lequel une diminution de la quantité de la 4EBP1 phosphorylée confirme l'inhibition de l'activité d'une kinase TOR chez le sujet.

2. Procédé in vitro selon la revendication 1, qui est un procédé pour déterminer une relation dose-réponse pour l'administration d'un inhibiteur de kinase TOR audit sujet, dans lequel on a administré audit sujet des doses variables dudit inhibiteur de kinase TOR et le niveau d'inhibition de l'activité d'une kinase TOR chez ledit sujet résultant de chaque dose dudit inhibiteur de kinase TOR est déterminé par utilisation de la cytométrie en flux pour mesurer la quantité de 4EBP1 phosphorylée présente dans des échantillons biologiques qui ont été obtenus auprès dudit sujet avant et après l'administration dudit inhibiteur de kinase TOR.

3. Procédé in vitro selon la revendication 1, qui est un procédé pour déterminer si ledit sujet est sensible à un inhibiteur de kinase TOR, dans lequel on a administré audit sujet ledit inhibiteur de kinase TOR et on détermine si oui ou non ladite kinase TOR est inhibée chez ledit sujet par utilisation de la cytométrie en flux pour mesurer la quantité de 4EBP1 phosphorylée présente dans des échantillons biologiques qui ont été obtenus auprès dudit sujet avant et après l'administration dudit inhibiteur de kinase TOR.

4. Procédé in vitro selon la revendication 1, qui est un procédé pour déterminer la quantité efficace d'un inhibiteur de kinase TOR pour le traitement ou la prise en charge d'une maladie chez un patient, dans lequel on a administré audit patient des doses variables dudit inhibiteur de kinase TOR et le niveau d'inhibition de l'activité d'une kinase TOR chez ledit patient résultant de chaque dose dudit inhibiteur de kinase TOR est déterminé par utilisation de la cytométrie en flux pour mesurer la quantité de 4EBP1 phosphorylée présente dans des échantillons biologiques qui ont été obtenus auprès dudit sujet avant et après l'administration dudit inhibiteur de kinase TOR.

5. Trousse comprenant un ou plusieurs contenants remplis d'un anticorps anti-p4EBP1, laquelle trousse est utilisée pour mesurer la quantité de 4EBP1 dans des échantillons biologiques au moyen de la cytométrie en flux, laquelle trousse comprend un inhibiteur de kinase TOR, l'inhibiteur de kinase TOR étant la 7-(6-(2-hydroxypropan-2-yl)pyridin-3-yl)-1-(t*rans*-4-méthoxycyclohexyl)-3,4-dihydropyrazino[2,3-b]pyrazin-2(1H)-one ou un sel pharmaceutiquement acceptable, clathrate, solvate, stéréoisomère, tautomère, ou promédicament de celle-ci.
